# FASCICULE DE BREVET EUROPEEN

(11) **EP 1 506 765 B1**
(45) Date de publication et mention de la délivrance du brevet: **25.07.2007**
(21) Numéro de dépôt: 04103850.6
(22) Date de dépôt: 10.08.2004
(51) Int. Cl.: A61K 8/04, A61K 8/11, A61K 8/19, A61K 8/84, A61Q 5/06

(54) **Composition cosmétique comprenant des particules metalliques stabilisées, eventuellement enrobées**
Kosmetiches Mittel enthaltend stabilisierte , gegebenfalls beschichtete, Metallteilchen
Cosmetic composition containing stabilized metallic particles, optionally coated

(30) Priorité: 11.08.2003 FR 0350421
(43) Date de publication de la demande: 16.02.2005
(73) Titulaire: L'ORÉAL, 75008 Paris (FR)
(72) Inventeur: GIROUD, Franck, 92110, CLICHY (FR)
(74) Mandataire: Poulin, Gérard

(56) Documents cités:
- EP-A- 1 034 839
- WO-A-97/06833
- WO-A-98/18867
- WO-A-02/064245
- WO-A-02/100371
- DATABASE CAPLUS [Online] XP002275041 extrait de STN Database accession no. 2002:764922
- DATABASE WPI Section Ch, Week 199923 Derwent Publications Ltd., London, GB; Class A60, AN 1999-267384 XP002275042 & JP 11 080647 A (NIPPON PAINT CO LTD) 26 mars 1999 (1999-03-26)

## Description

### DOMAINE TECHNIQUE

L'invention a trait à une composition cosmétique comprenant des particules métalliques stabilisées, lesdites particules pouvant éventuellement être enrobées et présenter une structure coeur-enveloppe (aussi appelées structure noyau-enveloppe ou "core-shell" en anglais).

L'invention concerne en outre lesdites particules stabilisées enrobées.

L'invention se rapporte également à un procédé cosmétique de traitement des matières kératiniques, telles que les cheveux, en particulier pour leur apporter de la brillance qui met en oeuvre ladite composition.

L'invention a trait en outre à l'utilisation de ladite composition pour apporter de la brillance aux matières kératiniques et en particulier aux cheveux.

Le domaine technique de l'invention peut être défini comme celui des compositions cosmétiques qu'il s'agisse de compositions capillaires ou de compositions pour la peau et les ongles.

L'utilisation de particules métalliques a déjà été décrite dans différents types de compositions cosmétiques de maquillage.

Ainsi, le document EP-A-1 082 952 décrit-il des compositions de maquillage, notamment des ongles, contenant des particules de verre recouvertes d'une couche métallique permettant l'obtention d'un maquillage présentant un aspect métallique éclatant et résistant à l'usure.

Le document EP-A-953 330, concerne l'association de deux compositions différentes comprenant respectivement des particules métalliques de type pigment goniochromatique et un pigment de type classique ayant une des couleurs du premier pigment pour l'obtention d'un maquillage à effet métallique variable selon l'angle d'observation et présentant des effets irisés.

Plus récemment, la demande de brevet internationale WO-A-02/03913 décrit des compositions de vernis à onges contenant des particules sous forme de plaquettes d'aluminium dans des proportions pondérales de 0,4 à 0,75% et des agents filmogènes ayant des poids moléculaires élevés pour l'obtention d'un maquillage de type miroir, c'est-à-dire en l'occurrence un maquillage ayant non seulement la couleur de l'aluminium mais également une brillance et une capacité à réfléchir les éléments distincts d'un objet.

Des particules métalliques ont également été incorporées dans des compositions capillaires.

On sait ainsi qu'il est possible d'apporter à la chevelure une brillance supérieure à celle apportée par les corps gras en incorporant dans les compositions capillaires des nanoparticules métalliques, en particulier des nanoparticules d'argent.

De telles compositions sont décrites dans le document EP-A-1 064 918.

Toutefois, il a été constaté que la brillance apportée par de telles compositions s'estompe très rapidement dans le temps.

Dans un autre domaine, le document WO-A-00 78282 décrit l'utilisation de nanoparticules d'argent d'une taille de 1 à 50 nm comme antimicrobien dans des compositions de caoutchouc silicone durcissable.

Ce document ne décrit pas toutefois ni la mise en oeuvre de nanoparticules encapsulées, ni la mise en oeuvre de nanoparticules stabilisées.

Il existe donc un besoin non encore satisfait pour une composition cosmétique et en particulier une composition cosmétique capillaire contenant des particules métalliques qui possède une brillance élevée, cette brillance étant maintenue sur une longue durée et ne s'estompant pas au cours du temps.

Il existe encore un besoin pour une composition cosmétique et en particulier une composition capillaire qui, tout en présentant une brillance élevée et sur une longue durée possède aussi une grande stabilité dans le temps.

Le but de la présente invention est de fournir une composition cosmétique qui réponde entre autres à ces besoins.

Le but de la présente invention est encore de fournir une composition cosmétique qui ne présente pas les inconvénients, défauts, limitations et désavantages des compositions de l'art antérieur et qui résolve les problèmes des compositions de l'art antérieur.

Ce but, et d'autres encore, sont atteints, conformément à l'invention par une composition cosmétique comprenant, dans un milieu physiologiquement acceptable, au moins un agent présentant une activité cosmétique et des particules, lesdites particules étant constituées en majorité par au moins un métal, lesdites particules étant des particules stabilisées, traitées par au moins un colloïde protecteur choisi parmi les polymères peignes comprenant une chaîne principale et des chaînes latérales reliées à la chaîne principale, et la taille desdites particules étant inférieure ou égale à 500 nm.

Des compositions cosmétiques contenant les particules spécifiques incorporées dans les compositions selon l'invention, qui sont définies par une structure spécifique, des constituants spécifiques en particulier en ce qui concerne l'agent stabilisant des particules, et une taille de particule spécifique, n'ont jamais été mentionnées dans l'art antérieur.

De manière étonnante, du fait de l'incorporation dans les compositions de l'invention de ces particules spécifiques, que l'on peut qualifier de nanoparticules métalliques stabilisées, les compositions de l'invention et en particulier des compositions capillaires permettent d'obtenir une brillance élevée immédiatement après leur application, c'est-à-dire juste après le traitement.

Mais, contrairement aux compositions de l'art antérieur qui contiennent des particules métalliques différentes de celles incorporées dans les compositions de l'invention, c'est-à-dire des particules métalliques non traitées (stabilisées) par des colloïdes protecteurs spécifiques, la brillance élevée obtenue avec les compositions de l'invention est conservée pendant une durée prolongée.

A titre d'exemple, cette brillance élevée peut être maintenue pendant une durée pouvant atteindre par exemple un mois ou plus pour des cheveux traités avec des compositions de l'invention alors qu'une composition de l'art antérieur telle que celle décrite dans le document EP-A-1 064 918 incluant des particules non-conformes à l'invention, à savoir non stabilisées, perd la totalité de sa brillance, de son pouvoir réflecteur après une durée de 1 mois.

Outre la conservation de la brillance dans le temps, les compositions cosmétiques selon l'invention présentent une stabilité dans le temps nettement supérieure à celle des compositions de l'art antérieur, représenté par exemple par le document EP-A-1 064 918, qui comprend des particules métalliques différentes de celles incluses dans les compositions de l'invention et, en particulier, non stabilisées par le colloïde protecteur spécifique selon l'invention.

Il semblerait, mais sans vouloir être lié par aucune théorie, que les particules métalliques spécifiques mises en oeuvre dans les compositions de l'invention limitent l'aggrégation des nanoparticules métalliques dans les milieux polaires comme l'eau et/ou l'éthanol et permettent ainsi d'obtenir des dispersions colloïdales d'une grande stabilité sans séparation de phases.

Les particules métalliques traitées (stabilisées) par un colloïde protecteur choisi parmi les polymères peignes qui sont mises en oeuvre dans les compositions de l'invention peuvent en outre, éventuellement être enrobées et présenter une structure coeur-enveloppe dans laquelle le coeur est constitué par la particule métallique stabilisée.

De telles particules, stabilisées par un colloïde protecteur choisi parmi les polymères peignes, et en outre enrobées n'ont jamais été décrites dans l'art antérieur et sont nouvelles.

L'incorporation de ces particules stabilisées et enrobées dans des compositions cosmétiques permet d'obtenir les effets de l'invention à un degré accru. Ainsi, en mettant en oeuvre des particules stabilisées enrobées, on obtient une brillance encore plus élevée pendant une durée encore plus longue et la stabilité des particules se trouve encore accrue.

L'invention a trait en particulier à une composition cosmétique capillaire, notamment à une composition cosmétique capillaire pour apporter aux cheveux de la brillance.

L'invention concerne en outre un procédé cosmétique de traitement des matières kératiniques telles que les cheveux en particulier pour apporter de la brillance aux matières kératiniques, en particulier aux cheveux, comprenant l'application sur les matières, fibres, kératiniques d'une composition telle que décrite ci-dessus.

L'invention a trait en outre à l'utilisation de la composition telle que décrite ci-dessus pour apporter de la brillance à des matières kératiniques telles que les cheveux.

L'invention concerne enfin l'utilisation des particules spécifiques décrites dans la présente, qu'elles soient stabilisées, ou stabilisées et enrobées, encapsulées, dans une composition cosmétique, pour apporter de la brillance aux matières, fibres kératiniques telles que les cheveux.

L'invention va maintenant être décrite de manière plus détaillée dans la description qui suit.

Les compositions cosmétiques selon l'invention comprennent au moins un agent présentant une activité cosmétique, ayant un effet cosmétique.

On entend par agent présentant une activité cosmétique ou actif cosmétique, au sens de la présente invention, tout composé actif ayant une activité cosmétique ou dermatologique ou encore tout composé susceptible de modifier l'aspect et/ou le toucher et/ou les propriétés physicochimiques des matières kératiniques telles que les cheveux.

Le ou les agent(s) présentant une activité cosmétique (le (ou les) actif(s) cosmétique(s)) est(sont) généralement choisi(s) parmi :
- les saccharides, les oligosaccharides, les polysaccharides hydrolysés ou non, modifiés ou non,
- les acides aminés, les oligopeptides, les peptides, les protéines hydrolysées ou non, modifiées ou non, les polyacides aminés, les enzymes,
- les acides et alcools gras ramifiés ou non,
- les cires animales, végétales ou minérales,
- les céramides et les pseudo-céramides,
- les acides organiques hydroxylés,
- les filtres UV,
- les antioxydants et les agents anti-radicaux libres,
- les chélatants,
- les agents antipelliculaires,
- les agents régulateurs de séborrée,
- les agents apaisants,
- les tensioactifs cationiques,
- les polymères cationiques, amphotères,
- les silicones organomodifiées ou non,
- les huiles minérales, végétales ou animales,
- les polyisobutènes et poly(α-oléfines),
- les esters,
- les polymères anioniques solubles ou dispersés,
- les polymères non-ioniques solubles ou dispersés,
- les agents réducteurs
- les agents colorants et matières colorantes, et notamment les colorants capillaires,
- les agents moussants,
- les agents filmogènes,
- les particules (autres que les particules stabilisées, éventuellement enrobées de l'invention),
- et leurs mélanges.

Cet agent présentant une activité cosmétique est présent dans une proportion de 0,001 à 10% en poids de la composition cosmétique, de préférence de 0,01 à 5% en poids.

D'une manière générale, les composés de type saccharides, oligosaccharides, polysaccharides hydrolysés ou non, modifiés ou non, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

A titre d'exemples de saccharides, oligosaccharides, polysaccharides hydrolysés ou non, modifiés ou non, utilisables dans l'invention, on peut notamment citer les glucanes, les amidons modifiés ou non (tels que ceux issus, par exemple, de céréales comme le blé, le maïs ou le riz, de légumes comme le pois blond, de tubercules comme les pommes de terre ou le manioc) et différents du bétaïnate d'amidon tel que décrit ci-dessus, l'amylose, l'amylopectine, le glycogène les dextranes, les β-glucanes, les celluloses et leurs dérivés (méthylcelluloses, hydroxyalkylcelluloses, éthylhydroxyéthylcelluloses, carboxyméthyl-celluloses), les fructosanes, l'inuline, la levane, les mannanes, les xylanes, les lignines, les arabanes, les galactanes, les galacturonanes, la chitine, les glucoronoxylanes, les arabinoxylanes, les xyloglucanes, les galactomannanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les gommes de guar et les gommes de xanthane.

Comme acides aminés, on peut citer, par exemple, la cystéine, la lysine, l'alanine, la N-phénylalanine, l'arginine, la glycine, la leucine, et leurs mélanges. A titre d'oligopeptides, de peptides, de protéines hydrolysées ou non, modifiées ou non, que l'on peut utiliser selon l'invention, on peut notamment citer les hydrolysats de protéines de laine ou de soie, modifiées ou non, les protéines végétales telles que les protéines de blé.

Parmi les polyacides aminés utilisables, on peut citer la polylysine.

Parmi les enzymes utilisables, on peut citer les laccases, les peroxydases, les lipases, les protéases, les glycosidases, les dextranases, les uricases, la phosphatase alcaline.

Parmi les acides gras ramifiés ou non convenant dans la présente invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges. Les alcools gras utilisables dans la présente invention, comprennent notamment les alcools en C₈-C₃₀ comme, par exemple, les alcools palmitylique, oléique, linoléique, myristylique, stéarylique et laurylique.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines ou les ozokérites.

Parmi les céramides, on peut citer en particulier les céramides des classes I, II, III et V selon la classification de DAWNING, et plus particulièrement la N-oléyldéshydrosphingosine.

Les acides organiques hydroxylés sont choisis parmi ceux bien connus et utilisés dans la technique. On peut notamment citer l'acide citrique, l'acide lactique, l'acide tartrique, l'acide malique.

Les filtres solaires actifs dans l'UV-A et/ou l'UV-B utilisables selon l'invention sont ceux bien connus de l'homme de métier. On peut notamment citer les dérivés du dibenzoylméthane tels que le 4-méthyldibenzoylméthane, le 4-isopropyldibenzoylméthane, le 4-tert-butyldibenzoylméthane, le 2,4-diméthyldibenzoylméthane, le 4-tert-butyl-4'-diisopropyldibenzoyl-méthane, l'acide p-aminobenzoïque et ses esters tels que le p-diméthylaminobenzoate de 2-éthylhexyle et le p-aminobenzoate d'éthyle N-propoxylé, les salicylates tels que le salicylate de triéthanolamine, les esters d'acide cinnamique tels que le 4-méthoxy-cinnamate de 2-éthylhexyle, le diisopropylcinnamate de méthyle, l'anthranilate de menthyle, les dérivés de benzotriazole, les dérivés de triazine, les dérivés de β,β-diphénylacrylate tels que le 2-cyano-3,3-diphénylacrylate de 2-éthylhexyle et le 2-cyano-3,3-diphénylacrylate d'éthyle, l'acide 2-phénylbenzimidazole-5-sulfonique et ses sels, les dérivés de benzophénone, les dérivés du benzylidène-camphre, les filtres siliconés, etc..

A titre d'antioxydants et d'agents anti-radicaux libres utilisables dans la présente invention, on peut citer, par exemple, l'acide ascorbique, des composés ascorbylés tels que le dipalmitate d'ascorbyle, la t-butylhydroquinone, les polyphénols tels que le phloroglucinol, le sulfite de sodium, l'acide érythorbique, les flavonoïdes.

Les chélatants peuvent être choisis notamment parmi l'EDTA (acide éthylènediaminetétraacétique) et ses sels tels que l'EDTA disodique et l'EDTA dipotassique, les composés phosphatés tels que le métaphosphate de sodium, l'hexamétaphosphate de sodium, le pyrophosphate tétrapotassique, les acides phosphoniques et leurs sels tels que les sels de l'acide éthylènediaminetétraméthylènephosphonique.

Les agents antipelliculaires sont choisis, par exemple, parmi :
- le chlorure de benzéthonium, le chlorure de benzalkonium, la chlorhexidine, la chloramine T, la chloramine B, la 1,3-dibromo-5,5-diméthylhydantoïne, la 1,3-dichloro 5,5-diméthylhydantoïne, la 3-bromo 1-chloro 5,5-diméthylhydantoïne, le N-chlorosuccinimide ;
- les dérivés de 1-hydroxy-2-pyridone tels que, par exemple, le 1-hydroxy-4-méthyl-2-pyridone, le 1-hydroxy-6-méthyl-2-pyridone et le 1-hydroxy-4,6-diméthyl-2-pyridone ;
- les trihalogénocarbamides ;
- le triclosan ;
- les composés azolés tels que le climbazole, le kétoconazole, le clotrimazole, l'econazole, l'isoconazole et le miconazole b ;
- les polymères antifongiques tels que l'amphotéricine B ou la nystatine ;
- les sulfures de sélénium ;
- le soufre sous ses différentes formes, le sulfure de cadmium, l'allantoïne, les goudrons de houille ou de bois et leurs dérivés en particulier, l'huile de cade, l'acide undécylénique, l'acide fumarique, les allylamines telles que la terbinafine.

On peut également les utiliser sous forme de leurs sels d'addition d'acides physiologiquement acceptables, notamment sous forme de sels d'acides sulfurique, nitrique, thiocyanique, chlorhydrique, bromhydrique, iodhydrique, phosphorique, acétique, benzoïque, glycolique, acéturique, succinique, nicotinique, tartrique, maléique, palmitique, méthane sulfonique, propanoïque, 2-oxopropanoïque, propanedioïque, 2-hydroxy-1,4-butanedioïque, 3-phényl-2-propènoïque, α-hydroxybenzèneacétique, éthanesulfonique, 2-hydroxyéthanesulfonique, 4-méthylbenzènesulfonique, 4-amino-2-hydroxybenzoïque, 2-phénoxybenzoïque, 2-acétyloxybenzoïque, picrique, lactique, citrique, malique, oxalique et d'aminoacides.

Les agents antipelliculaires mentionnés ci-dessus peuvent aussi le cas échéant être utilisés sous forme de leurs sels d'addition de bases organiques ou inorganiques physiologiquement acceptables. Des exemples de bases organiques sont notamment les alcanolamines de faibles poids moléculaires telles que l'éthanolamine, la diéthanolamine, la N-éthyléthanolamine, la triéthanolamine, le diéthylaminoéthanol, le 2-amino-2-méthylpropanedione; les bases non-volatiles telles que l'éthylènediamine, l'hexaméthylènediamine, la cyclohexylamine, la benzylamine, la N-méthylpipérazine; les hydroxydes d'ammonium quaternaires, par exemple, l'hydroxyde de triméthylbenzylammonium ; la guanidine et ses dérivés, et particulièrement ses dérivés alkylés. Des exemples de bases inorganiques sont notamment les sels de métaux alcalins, comme le sodium ou potassium; les sels d'ammonium, les sels de métaux alcalino-terreux, comme le magnésium, calcium ; les sels de métaux di, tri ou tétravalents cationiques, comme le zinc, l'aluminium et le zirconium. Les alcanolamines, l'éthylènediamine et les bases inorganiques telles que les sels de métaux alcalins sont préférées.

Les agents régulateurs de séborrhée sont par exemple le succinylchitosane et la poly-β-alanine.

Les agents apaisants sont par exemple l'azulène et l'acide glycyrrhétinique.

Les tensioactifs cationiques sont ceux bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyl-trialkylammonium, de trialkylbenzylammonium, de trialkylhydroxyalkylammonium ou d'alkylpyridinium ; les dérivés d'imidazoline.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français nos 2 505 348 et 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyltriméthylammonium, de méthacrylamidopropyltriméthylammonium ou de diméthyldiallylammo-nium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique est compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammo-nium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206.462, 2 261 002, 2 271 378, 3.874.870, 4.001.432, 3 929 990, 3 966 904, 4 005 193, 4 025 617, 4 025 627, 4 025 653, 4 026 945 et 4 027 020 ;
(11) les polymères de polyammoniums quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat^{®} FC 905, FC 550 et FC 370 par la société B.A.S.F ;
(13) les polyamines comme le Polyquart^{®} H vendu par HENKEL, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄) ammonium tels que ceux commercialisés sous les noms de SALCARE^{®} SC 92, SALCARE^{®} SC 95 et SALCAREOE^{®} SC 96 par la Société ALLIED COLLOIDS ; et
leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères amphotères utilisables conformément à l'invention peuvent être choisis parmi les polymères comportant des motifs B et C répartis statistiquement dans 1a chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylates et acrylates, les dialkylaminoalkyl-méthacrylamides et acrylamides. De tels composés sont décrits dans le brevet américain n° 3 836 537. On peut également citer le copolymère acrylate de sodium/chlorure d'acrylamidopropyltriméthylammonium vendu sous la dénomination POLYQUART^{®} KE 3033 par la Société HENKEL.
   Le composé vinylique peut être également un sel de dialkyldiallylammonium tel que le chlorure de diéthyldiallyl-ammonium.
   Les copolymères d'acide acrylique et de ce dernier monomère sont proposés sous les appellations MERQUAT^{®} 280, MERQUAT^{®} 295 et MERQUAT^{®} PLUS 3330 par la société CALGON.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide. le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/acrylates/butylaminoethylmethacrylate copolymer tels que les produits vendus sous la dénomination AMPHOMER^{®} ou LOVOCRYL^{®} 47 par la société NATIONAL STARCH.
(3) les polyaminoamides réticulés et alkylés partiellement ou totalement dérivant de polyaminoamides de formule générale : 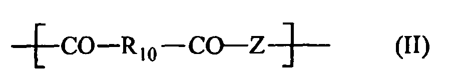 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 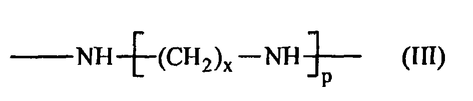 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylènediamine, ou le groupe dérivant de la pipérazine 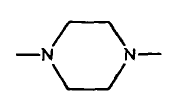
   c) dans les proportions de 0 à 20 % en moles, le groupe -NH-(CH₂)₆-NH- dérivant de l'hexaméthylènediamine, ces polyaminoamides étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 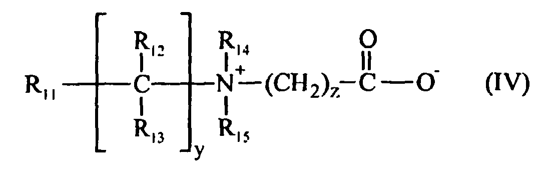 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupement méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
   A titre d'exemple, on peut citer le copolymère de méthacrylate de méthyle et de méthacrylate de diméthyl-carboxyméthylammonio-éthyle tel que le produit vendu sous la dénomination DIAFORMER^{®} Z301 par la société SANDOZ.
(5) les polymères dérivés du chitosane comportant des motifs monomères répondant aux formules suivantes: 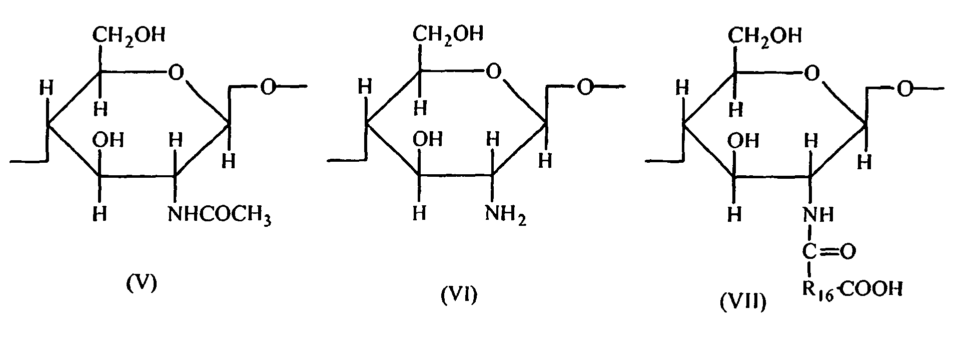 le motif (V) étant présent dans des proportions comprises entre 0 et 30 %, le motif (VI) dans des proportions comprises entre 5 et 50% et le motif (VII) dans des proportions comprises entre 30 et 90%, étant entendu que dans ce motif (VII), R₁₆ représente un groupe de formule: 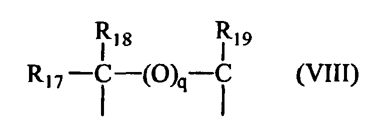 dans laquelle si q=0, R₁₇, R₁₈ et R₁₉, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ; ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl-chitosane ou le N-carboxybutylchitosane vendu sous la dénomination EVALSAN^{®} par la société JAN DEKKER.
(7) Les polymères répondant à la formule générale (IX) décrits par exemple, dans le brevet français 1 400 366: 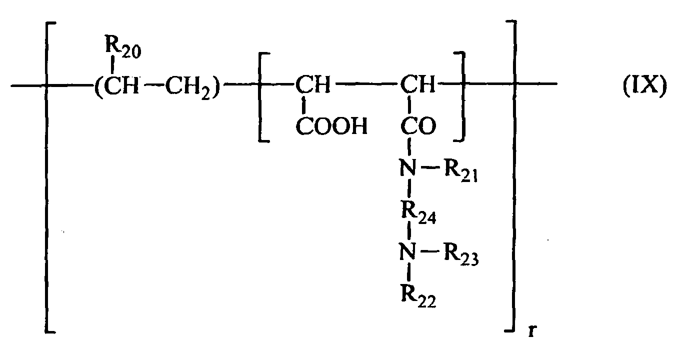 dans laquelle R₂₀ représente un atome d'hydrogène, un groupe CH₃O, CH₃CH₂O, phényle, R₂₁ désigne l'hydrogène ou un groupe alkyle inférieur tel que méthyle, éthyle, R₂₂ désigne l'hydrogène ou un groupe alkyle inférieur tel que méthyle, éthyle, R₂₃ désigne un groupe alkyle inférieur tel que méthyle, éthyle ou un groupe répondant à la formule: -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus, ainsi que les homologues supérieurs de ces groupes et contenant jusqu'à 6 atomes de carbone.
(8) Des polymères amphotères du type -D-X₁-D-X₁- choisis parmi:
   a) les polymères obtenus par action de l'acide chloracétique ou le chloracétate de sodium sur les composés comportant au moins un motif de formule:

      -D-X₁-D-X₁-D- (X)

      où D désigne un groupe 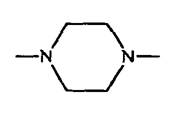 et X₁ désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X₁-D-X₁- (XI)

      où D désigne un groupe 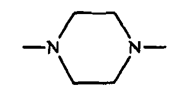 et X₁ désigne le symbole E ou E' et au moins une fois E'; E ayant la signification indiquée ci-dessus et E' étant un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.

Les silicones utilisables conformément à l'invention peuvent être solubles ou insolubles dans l'eau, et elles peuvent être en particulier des polyorganosiloxanes insolubles dans l'eau ; elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA.
   On peut également citer les cyclocopolymères du type diméthylsiloxane/méthylalkylsiloxane, tel que la "SILICONE VOLATILE FZ 3109" commercialisée par la société UNION CARBIDE, de structure chimique
   avec D : 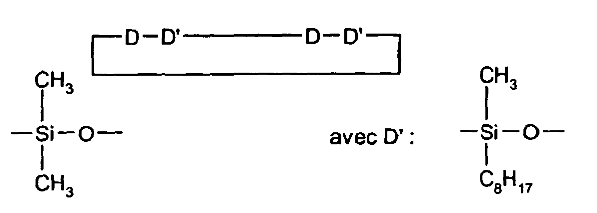
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous les dénominations GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous les dénominations "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorgano-siloxanes décrits dans la demande EP 342 834. On peut citer, par exemple, le produit Q2-8413 de la société DOW CORNING.

On peut notamment citer comme huiles d'origine végétale, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; comme huile d'origine animale, le perhydrosqualène ; comme huiles d'origine minérale, l'huile de paraffine et l'huile de vaseline.

Les polyisobutènes et poly(α-oléfines) sont choisis parmi ceux bien connus dans la technique.

Comme exemples d'esters, on peut notamment citer les esters d'acides gras comme le myristate d'isopropyle, le palmitate d'iso-propyle, le palmitate de 2-éthylhexyle, l'huile de Purcellin (octanoate de stéaryle), l'isononanoate d'isononyle ou d'isostéaryle, le lanolate d'isopropyle, et leurs mélanges.

Les polymères anioniques généralement utilisés dans la présente invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 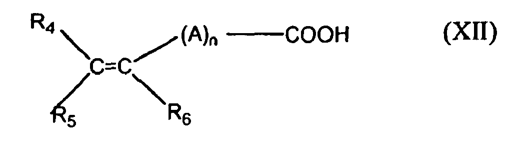 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₄ désigne un atome d'hydrogène, un groupement phényle ou benzyle, R₅ désigne un atome d'hydrogène, un groupement alkyle inférieur ou carboxyle, R₆ désigne un atome d'hydrogène, un groupement alkyle inférieur, un groupement -CH₂-COOH, phényle ou benzyle.

Dans la formule (XII) ci-dessus, un groupement alkyle inférieur comporte de préférence de 1 à 4 atomes de carbone et désigne en particulier, les groupements méthyle et éthyle.

Les polymères anioniques à groupements carboxyliques préférés selon l'invention sont :
A) les homo- ou copolymères d'acide acrylique ou méthacrylique ou leurs sels, et en particulier les produits vendus sous les dénominations VERSICOL^{®} E ou K par la société ALLIED COLLOID, ULTRAHOLD^{®} par la société BASF Les copolymères d'acide acrylique et d'acrylamide vendus sous la forme de leur sel de sodium sous les dénominations RETEN^{®} 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER^{®} par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER^{®} MAEX par la société BASF.
C) les copolymères dérivés d'acide crotonique tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ^{®} AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène-sulfonique, les sels de sodium, ayant une masse moléculaire d'environ 500 000 et d'environ 100 000 vendus respectivement sous les dénominations Flexan^{®} 500 et Flexan^{®} 130 par National Starch. Ces composés sont décrits dans le brevet FR 2198719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER^{®} HSP 1180 par Henkel.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG^{®} par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique monoestérifié vendu sous la dénomination GANTREZ^{®} ES 425 par la société ISP, les copolymères d'acide méthacrylique et de méthacrylate de méthyle vendus sous la dénomination EUDRAGIT^{®} L par la société ROHM PHARMA, le copolymère d'acide méthacrylique et d'acrylate d'éthyle vendu sous la dénomination LUVIMER^{®} MAEX par la société BASF, le copolymère acétate de vinyle/acide crotonique vendu sous la dénomination LUVISET^{®} CA 66 par la société BASF et le terpolymère acétate de vinyle/acide crotonique/polyéthylèneglycol vendu sous la dénomination ARISTOFLEX^{®} A par la société BASF.

Selon l'invention, on peut également utiliser les polymères anioniques sous forme de latex ou de pseudolatex, c'est-à-dire sous forme d'une dispersion aqueuse de particules de polymères insolubles.

À titre de polymères non ioniques utilisables selon la présente invention, on peut notamment citer :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX^{®} 50 000, PEOX^{®} 200 000 et PEOX^{®} 500 000 ;
- les homopolymères d'acétate de vinyle tels que le produit proposé sous le nom de APPRETAN^{®} EM par la société HOECHST ou le produit proposé sous le nom de RHODOPAS^{®} A 012 par la société RHONE POULENC ;
- les copolymères d'acétate de vinyle et d'ester acrylique tels que le produit proposé sous le nom de RHODOPAS^{®} AD 310 de RHONE POULENC ;
- les copolymères d'acétate de vinyle et d'éthylène comme le produit proposé sous le nom de APPRETAN^{®} TV par la société HOECHST;
- les copolymères d'acétate de vinyle et d'ester maléique, par exemple de maléate de dibutyle tels que le produit proposé sous le nom de APPRETAN^{®} MB EXTRA par la société HOECHST ;
- les copolymères de polyéthylène et d'anhydride maléique ;
- les homopolymères d'acrylates d'alkyle et les homopolymères de méthacrylates d'alkyle tels que le produit proposé sous la dénomination MICROPEARL^{®} RQ 750 par la société MATSUMOTO ou le produit proposé sous la dénomination LUHYDRAN^{®} A 848 S par la société BASF ;
- les copolymères d'esters acryliques tels que, par exemple, les copolymères d'acrylates d'alkyle et de méthacrylates d'alkyle comme les produits proposés par la société ROHM & HAAS sous les dénominations PRlMAL^{®} AC-261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAW^{®} N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL^{®} LX 531 8 par la société NIPPON ZEON ou ceux proposés sous la dénomination CJ 0601 8 par la société ROHM & HAAS ;
- les polyuréthannes tels que les produits proposés sous les dénominations ACRYSOL^{®} RM 1020 ou ACRYSOL^{®} RM 2020 par la société ROHM & HAAS, les produits URAFLEX^{®} XP 401 UZ, URAFLEX^{®} XP 402 UZ proposé par la société DSM RESINS ;
- les copolymères d'acétate d'alkyle et d'uréthanne tels que le produit 8538-33 par la société NATIONAL STARCH ;
- les polyamides tels que le produit ESTAPOR^{®} LO 11 proposé par la société RHONE POULENC ;
- les gommes de guar non ioniques chimiquement modifiées ou non modifiées.

Les gommes de guar non ioniques non modifiées sont, par exemple, les produits vendus sous la dénomination VIDOGUM^{®} GH 175 par la société UNIPECTINE et sous la dénomination JAGUAE^{®} C par la société MEYHALL.

Les gommes de guar non-ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont, par exemple, vendues sous les dénominations commerciales JAGUAR^{®} HP8, JAGUAR^{®} HP60 et JAGUAR^{®} HP120, JAGUAR^{®} DC 293 et JAGUAR^{®} HP 105 par la société MEYHALL, ou sous la dénomination GALACTASOL^{®} 4H4FD2 par la société AQUALON.

Les groupes alkyle des polymères non ioniques comportent de préférence de 1 à 6 atomes de carbone.

Les agents réducteurs peuvent être choisis parmi les thioacides et leurs sels (acide thioglycolique ou thiosulfate, cystéine ou cystéamine), les sulfites de métaux alcalins ou alcalinoterreux, les sucres réducteurs tels que le glucose, la vitamine C et ses dérivés, les dérivés d'acide sulfovinique, les phosphines.

Les agents colorants peuvent être choisis parmi les structures conjuguées linéaires ou aromatiques (hétérocycliques ou non). Parmi celles-ci, on peut citer les colorants benzéniques nitrés, les colorants aromatiques, les colorants amino-benzéniques, les colorants azoïques, les colorants anthraquinoniques, les diamines aromatiques, les aminophénols, les phénols et naphtols, les porphyrines, tétraphénylporphyrines, métalloporphyrines, les phtalocyanines, les caroténoïdes, les flavonoïdes, les molécules fluorescentes (fluoresceine, rhodamine, coumarine ...).

Les agents filmogènes peuvent être choisis parmi les polymères filmogènes, par exemple ceux décrits dans FR-2 739 022, FR-2 757 048 ou FR-2 767 699 appartenant à l'Oréal.

Les agents moussants peuvent être choisis généralement parmi les tensioactifs au caractère moussant, les polymères cationiques et les polymères anioniques aux propriétés moussantes ; ou encore, il peut s'agir d'un agent spécifique tel que celui décrit dans FR-2 751 221 appartenant à l'Oréal.

Les particules, en tant qu'actifs cosmétiques, sont autres que les particules à structure coeur-enveloppe de l'invention et peuvent être choisis parmi les particules organiques, minérales ou en composite.

Les compositions cosmétiques selon l'invention sont essentiellement caractérisées par les particules qu'elles contiennent. Ces particules sont selon l'invention des particules qui peuvent être définies comme étant des nanoparticules métalliques, traitées, stabilisées par un colloïde protecteur et éventuellement encapsulées par, par exemple, un matériau organique.

Au sens de la présente invention, on entend par "nanoparticules" des particules dont la taille est inférieure ou égale à 500 nm, de préférence comprise entre 1 nm et 500 nm, de préférence encore comprise entre 1 nm et 100 nm, mieux de 1 nm à 50 nm.

On entend par "taille de particule" la dimension maximale qu'il est possible de mesurer entre deux points de la particule. De telles tailles sont mesurables directement par des techniques de microscopie comme la microscopie à balayage électronique ou la microscopie à force atomique, ou par des techniques indirectes comme la diffusion dynamique de la lumière.

Les particules incorporées dans les compositions de l'invention peuvent avoir des formes variées, elles peuvent revêtir par exemple la forme de sphères, de lamelles, de fibres, de tubes, de polyèdres, elles peuvent aussi avoir une forme totalement aléatoire. Une forme préférée est sphérique.

Les particules stabilisées incorporées dans les compositions de l'invention sont constituées en majorité par un métal.

Par métal, on entend un corps simple constitué uniquement d'atomes d'un élément métallique susceptible d'engendrer des cations.

Dans le cas où les particules stabilisées sont enrobées, encapsulées, et possèdent une structure coeur-enveloppe ("core-shell") la particule stabilisée constitue le coeur de la particule stabilisée, enrobée à structure coeur-enveloppe et ce coeur, noyau ("core") est donc constitué en majorité par au moins un métal.

Par "en majorité", on entend que la particule est constituée par 50% ou plus en masse d'au moins un métal.

De préférence, la particule est constituée par au moins 80% en masse, de préférence encore par au moins 90%, en masse et mieux par 100% en masse d'au moins un métal.

On entend généralement par métal, l'aluminium et tous les éléments de numéro atomique allant de 21 à 82 et composant les colonnes 3 à 13 de la classification périodique des éléments selon la nouvelle notation IUPAC : on pourra à ce sujet se référer au "CRC Handbook of Chemistry and Physics", 80th Print Edition.

Le terme métal inclut également tous les alliages de ces éléments, et les mélanges de ces métaux et alliages.

La particule peut, ainsi également, être constitué dans les pourcentages cités ci-dessus par un mélange de deux ou plus de ces métaux et/ou alliages de ceux-ci.

La particule peut aussi être une particule composite constitué de plusieurs zones, des zones adjacentes étant constituées par des métaux, alliages ou mélanges différents.

Des particules composites préférées sont des particules multicouches comprenant un coeur ou noyau interne formant substrat constitué par un métal, alliage ou mélange de métaux ou d'alliages recouvert au moins partiellement par une première couche en un métal, alliage de métal ou mélange de métaux ou d'alliages différent de celui constituant le coeur ou noyau interne, et éventuellement par une ou plusieurs autres couches, chacune de ces couches recouvrant au moins partiellement la couche précédente et chaque couche étant constituée par un métal, alliage ou mélange de métaux ou d'alliages différent de la couche suivante (si celle-ci existe) et de la couche précédente.

Outre ledit métal ou lesdits métaux, la particule peut comprendre en outre des impuretés inévitables, et des stabilisants de toute sorte.

La particule peut aussi comprendre par exemple en outre des composés métalliques autres que des métaux tels que des oxydes de métaux.

Ainsi, dans le cas de l'aluminium, la particule peut comprendre de l'alumine Al₂O₃ dans une proportion par exemple de 10%, pour 90% de Al métal.

Le métal qui constitue en majorité des particules est choisi de préférence parmi les métaux de transitions, les métaux des terres rares et leurs alliages et mélanges.

De préférence encore, le métal est choisi parmi l'aluminium, le cuivre, l'argent, l'or, l'indium, le fer, le platine, le nickel, le molybdène, le titane, le tungstène, l'antimoine, le palladium, le zinc, l'étain et leurs alliages et mélanges.

Parmi la liste précédente, les métaux dits "nobles" et le cuivre sont préférés. On entend par métaux nobles, l'or, l'argent, le palladium, le platine et leurs alliages et mélanges.

L'argent est le métal préféré entre tous.

En effet, selon l'invention, la perte de brillance, due très probablement à l'oxydation superficielle de la fine couche d'argent déposée à la surface d'une fibre kératinique est évitée grâce à la protection, à la stabilisation, procurée par le colloïde protecteur spécifique. Cette protection, stabilisation est accrue lorsqu'on met en oeuvre comme décrit plus loin des particules enrobées, comportant une enveloppe entourant un coeur formé par la particule stabilisée.

Par polymère peigne, on entend un polymère comprenant une chaîne principale ou squelette et des greffons ou chaînes latérales reliés à la chaîne principale.

Le polymère peigne a de préférence une chaîne principale ou squelette choisie parmi les polyalkylène imines.

Généralement, les chaînes latérales ou greffons sont choisies parmi les chaînes latérales ou greffons présentant de l'affinité avec le milieu physiologiquement acceptable, c'est-à-dire le milieu de dispersion ; de préférence les greffons sont hydrophiles.

Les chaînes latérales ou greffons sont de préférence choisis parmi les polyesters, les polyuréthanes, les acides poly(méth)acryliques, les poly(méth)acrylates et tous les homopolymères ou copolymères présentant de l'affinité avec le milieu physiologiquement acceptable utilisé.

Pour plus de détails sur la chimie et les propriétés de ces colloïdes protecteurs pour particules métalliques, on pourra se référer aux documents suivants :
- Kagaku Kogyo (2001), 52(3), 209-214,
- Kagaku Kogyo (2000), 53(8), 909-911,
- Document JP-A-11 80647.

Les particules stabilisées sont de préférence susceptibles d'être préparées par réduction d'un ou de plusieurs composés métalliques précurseurs du ou des métaux constituant la particule, en présence du polymère peigne.

La réduction peut être effectuée dans un solvant, de préférence un solvant organique et/ou de l'eau, dans lequel on dissout le ou les composé(s) métallique(s) précurseur(s), et auquel on ajoute le polymère peigne puis un réducteur.

Le ou les composé (s) métallique (s) précurseur(s) peut (peuvent) être choisi(s) par exemple parmi l'acide chloroaurique, le nitrate d'argent, l'acétate d'argent, l'acide d'argent perchlorique, l'acide chloroplatinique, le chloroplatinate de potassium.

Le réducteur est de préférence choisi parmi les réducteurs doux, par exemple parmi les molécules possédant une amine secondaire ou une amine tertiaire tels que le 2-méthylaminoéthanol, la diéthanolamine, la diéthylméthylamine, le 2-diméthylaminoéthanol, et la diéthylméthylamine.

Pour plus de détails sur un tel procédé, on pourra se reporter aux documents suivants :
- Kagaku Kogyo (2001), 52(3), 209-214 ;
- Kagaku Kogyo (2000), 53(8), 909-911 ;
- Document JP-A-11 080647.

Avantageusement, les particules incorporées dans les compositions cosmétiques selon l'invention sont des particules enrobées qui comprennent un coeur et une enveloppe solide (structure "core-shell"), le coeur étant constitué en majorité par au moins un métal, et étant traité (stabilisé) par un colloïde protecteur choisi parmi les polymères peignes, ledit coeur étant entouré, enrobé, encapsulé par une enveloppe solide, et la taille desdites particules étant comprise entre 1 nm et 500 nm.

De telles particules stabilisées et enrobées n'ont jamais été décrites dans l'art antérieur et accroissent encore de manière surprenante les effets de l'invention.

L'enveloppe peut être constituée par un matériau organique ou bien par un matériau inorganique lié au coeur par une liaison non covalente.

Selon une première forme de réalisation de l'invention, l'enveloppe entourant le coeur est constituée par un matériau organique.

Par matériau organique, on entend également tous les matériaux comprenant au moins un atome de carbone.

Selon l'invention, ce matériau est un matériau solide à température ambiante et à pression atmosphérique.

Le matériau organique peut ainsi être choisi parmi les polymères ou oligomères organiques, qu'il s'agisse de composés naturels ou synthétiques.

Les polymères ou oligomères de l'invention peuvent être obtenus notamment par polymérisation radicalaire ou par polycondensation.

Le matériau organique pourra ainsi être choisi parmi les poly(alcool vinylique), les poly(acide lactique), les poly(acide glycolique), les copolymères de l'acide lactique et de l'acide glycolique, les polystyrènes, les poly(méth)acrylates de méthyle, les copolymères acryliques et méthacryliques, les polyamides, les polyesters, les polyuréthanes, et les polyurées.

Le matériau organique peut aussi être choisi parmi la cellulose et les dérivés de celle-ci comme les alkyl et hydroxyalkylcelluloses telles que la méthylcellulose, l'éthylcellulose et l'hydroxyéthyl cellulose, ou comme les esters de cellulose tels que l'acétophtalate de cellulose.

Le matériau organique peut enfin être choisi parmi la gélatine, la pectine, éventuellement réticulées par exemple avec le glutaraldéhyde ou un polysaccharide tel que le carraghénane.

Un matériau organique préféré est un copolymère styrène-acide méthacrylique.

Selon une seconde forme de réalisation de l'invention, l'enveloppe contenant le coeur est constituée par un matériau inorganique choisi parmi les oxydes métalliques et les polymères organométalliques.

L'enveloppe, la capsule constituée par un matériau organique ou inorganique a généralement une épaisseur de 2 à 300 nm, de préférence de 5 à 250 ; de préférence encore de 10 à 100.

Il est à noter que cette enveloppe, cette capsule, et conformément à la définition bien connue de l'encapsulation dans le domaine de la technique, n'est pas une monocouche, une couche moléculaire mais bien une couche que l'on peut qualifier de paroi "épaisse" dont l'épaisseur est généralement comprise dans la plage définie ci-dessus.

Selon l'invention, la capsule, enrobage ou enveloppe, en matériau organique ou inorganique est reliée au noyau ou coeur par une liaison physique, sans liaisons covalentes. En d'autres termes, dans les particules incorporées dans les compositions de l'invention, l'interface noyau/enveloppe est définie comme ne présentant pas de liaisons covalentes.

La formation de l'enveloppe, de la capsule, en matériau organique autour du noyau métallique, (constitué par la particule métallique stabilisée par le colloïde protecteur) dans les particules enrobées des compositions de l'invention peut être réalisée par divers procédés.

Ces procédés qui sont généralement désignés par les termes de procédé d'encapsulation ou de nanoencapsulation sont connus de l'homme du métier dans ce domaine de la technique et peuvent être généralement divisés en deux grandes familles : à savoir d'une part les procédés physico-chimiques et d'autre part les procédés chimiques.

Les procédés physico-chimiques peuvent être choisis parmi la séparation de phase ou coacervation, l'évaporation, l'extraction de solvant, la gélification thermique, la précipitation contrôlée, et tous les autres procédés physico-chimiques de microencapsulation connus.

Les procédés chimiques peuvent être choisis parmi la polycondensation interfaciale, la polymérisation interfaciale, la polymérisation en milieu dispersé, la polycondensation in-situ, la polymérisation en émulsion, et tous les autres procédés chimiques de microencapsulation connus.

Pour les procédés physico-chimiques et chimiques, on pourra aussi se reporter au document "Microencapsulation Methods and Industrial Applications", (ISBN 0-8247-9703-S).

Parmi les divers procédés d'encapsulation utilisable, on préfère l'encapsulation par polymérisation en émulsion et encore plus spécifiquement l'encapsulation par polymérisation en émulsion telle que décrite dans l'article "Preparation of Polymer Coated Fonctionalised Silver Nanoparticles" (J. Am. Chem. Soc. 1999, 121, 10642-10643).

Telle qu'elle est décrite dans le précédent article, l'encapsulation des nanoparticules est obtenue par un classique procédé de polymérisation radicalaire en émulsion. Toutefois, afin d'obtenir une polymérisation spécifique sur la surface des nanoparticules métalliques, la phase micellaire de l'émulsion est constituée par un composé amphiphile ou un mélange de composés amphiphiles dont la partie polaire ou la partie hydrophobe est affine avec la surface métallique.

Un tel procédé permet d'obtenir un système noyau (coeur)-enveloppe ("core shell") constitué par un noyau métallique (constitué par une particule métallique stabilisée par un colloïde protecteur) et une capsule de polymère d'une épaisseur supérieure à 2 nm et classiquement comprise entre 2 nm et 300 nm.

Parmi les divers constituants exploitables pour réaliser la polymérisation en émulsion décrite précédemment sont préférés :
- l'eau et/ou l'éthanol ou leurs mélanges comme phase continue de l'émulsion, l'eau étant préférée.
- Les molécules amphiphiles dont la partie polaire est constituée par une ou plusieurs fonctions carboxyliques. Parmi les molécules amphiphiles sont préférées les acides gras et plus particulièrement l'acide oléique.
- Un mélange de styrène et d'acide méthacrylique comme monomères précurseurs de la capsule.
- Des nanoparticules d'argent stabilisées par une polyéthylène imine à greffons hydrophiles comme support à encapsuler.

La surface externe des particules stabilisées et enrobées, c'est-à-dire la surface externe de la capsule, de l'enveloppe, peut être modifiée de façon covalente par au moins un groupement chimique qui est susceptible d'améliorer l'adsorption des particules sur les matières kératiniques telles que les cheveux. Cette surface peut être modifiée aussi de façon covalente par au moins un groupement chimique capable de réagir chimiquement avec les matières kératiniques telles que les cheveux.

Dans le premier cas, l'adsorption sur les matières kératiniques telles que les cheveux des nanoparticules "core shell" des compositions de l'invention peut être améliorée en modifiant de façon covalente la capsule de matériau organique ou inorganique tel qu'un polymère par différents groupements chimiques (groupe A ci-dessous) rendant la surface des particules par exemple, plus hydrophobe, et/ou plus cationique, et/ou plus anionique, et/ou plus hydrophile.

On définit l'adsorption comme mettant en oeuvre des énergies de liaisons plus faibles que des liaisons covalentes, c'est-à-dire inférieure à 50 kcal/mol entre la matière kératinique telle que le cheveu et la particule. Ces liaisons de faibles énergies sont par exemple les forces de Van der Waals, liaisons hydrogènes, complexes donneur-accepteur d'électrons, etc...

Le groupement capable d'améliorer l'adsorption des particules sur les matières kératiniques est généralement choisi parmi les groupements du groupe A suivant :

### Groupe A :

- Acides carboxyliques et leurs sels,
- Amines primaires, secondaires, tertiaires et quaternaires,
- Phosphates,
- Oxydes de soufre tels que sulfones, sulfoniques, sulfoxides, et sulfates,
- Noyaux aromatiques tels que phényle, triazine, thiophène et imidazole.

Dans le second cas, l'adhésion sur les matières kératiniques telles que les cheveux des nanoparticules de l'invention est obtenue en modifiant de façon covalente la capsule de matériau organique ou inorganique, par exemple de polymère par différents groupements (groupe B) capables de réagir chimiquement sur la matière kératinique. Plus spécifiquement, on entend par groupements possédant une réactivité sur la matière kératinique en particulier le cheveu, les groupements capables de former une liaison covalente avec cette matière par exemple avec les amines et/ou les acides carboxyliques et/ou les thiols des acides aminés constituant la matière kératinique. La formation de ces liaisons covalentes peut être soit spontanée, soit se produire par une activation par la température, le pH, la lumière, un co-réactif, ou par un catalyseur chimique ou biochimique tel qu'un enzyme.

Le groupement susceptible, capable de réagir chimiquement avec les matières kératiniques telles que les cheveux est généralement choisi parmi les groupements du groupe B suivant :

### Groupe B :

- Epoxydes,
- Vinyle et vinyle activé : acrylonitrile, esters acrylique et méthacrylique, acide et esters crotonique, acide et esters cinnamique, styrène et dérivés, butadiène, éthers de vinyle, vinyle cétone, esters maléique, maléimides, vinyl sulfones, ...
- Acides carboxyliques et leurs dérivés : anhydride, chlorure d'acide, esters,
- Acétals, hémi-acétals,
- Aminals, hémi-aminals,
- Cétones et alpha-hydroxycétones, alpha-halocétones,
- Lactones, thiolactones,
- Isocyanates,
- Thiocyanates,
- Imines,
- Imides (succinimides, glutimides),
- Pyridyldithio,
- N hydroxysuccinimide esters,
- Imidates,
- Oxazine et oxazoline,
- Oxazinium et oxazolinium,
- Groupements de formule R₁X, dans laquelle R₁ est un groupe alkyle de 1 à 30C, un groupe aryle comprenant de 6 à 30C, un groupe aralkyle de 7 à 30C, (groupe alkyle de 1 à 30C) et X est un groupe partant tel que I, Br, Cl, OSO₃R où R est H, un groupe alkyle de 1 à 30C, -SO₂R' où R' est H, un groupe alkyle de 1 à 30C, un groupe tosyle, N(R")₃ où R" est un groupe alkyle de 1 à 30C, OPO₃R'"₂ où R'" est H ou un groupe alkyle de 1 à 30C ; des groupements de formule R₁X préférés sont les halogénures d'alkyle, d'aryle ou d'aralkyle ;
- Groupements de formule R₂X où R₂ représente un cycle carboné de 3 à 30C ou un hétérocycle insaturé de 3 à 20 chaînons comprenant un ou plusieurs hétéroatomes choisis parmi N, S, O et P, et X est un groupe partant tel que défini ci-dessus ; des groupements de formule R₂X préférés sont les halogénures de cycles insaturés tels que la chlorotriazine, la chloropyrimidine, la chloroquinoxaline, et le chlorobenzotriazole,
- Groupements de formule : R₃SO₂X où R₃ a la même signification que R₁ et X représente un groupe partant et a la signification déjà donnée ci-dessus,
- Lactones,
- Thiolactones,
- Siloxanes.

A titre d'exemple, de façon non exhaustive, on peut citer l'activation par le N-hydroxysulfosuccinimide de particles coeur/enveloppe (coquille) ("core/shell") de type argent/copolymère styrène méthacrylique. Les fonctions sulfosuccinimides sont dans le cas d'une enveloppe, capsule organique greffées à la surface des particules par l'intermédiaire des groupements carboxyliques que possède le polymère de la capsule. Un tel groupement de surface permet de lier de façon covalente les nanoparticules de l'invention aux cheveux par reaction sur les amines libres superficielles que possède la fibre capillaire (voir schéma réactionnel ci-dessous).

### 1- Activation des nanoparticules encapsulées

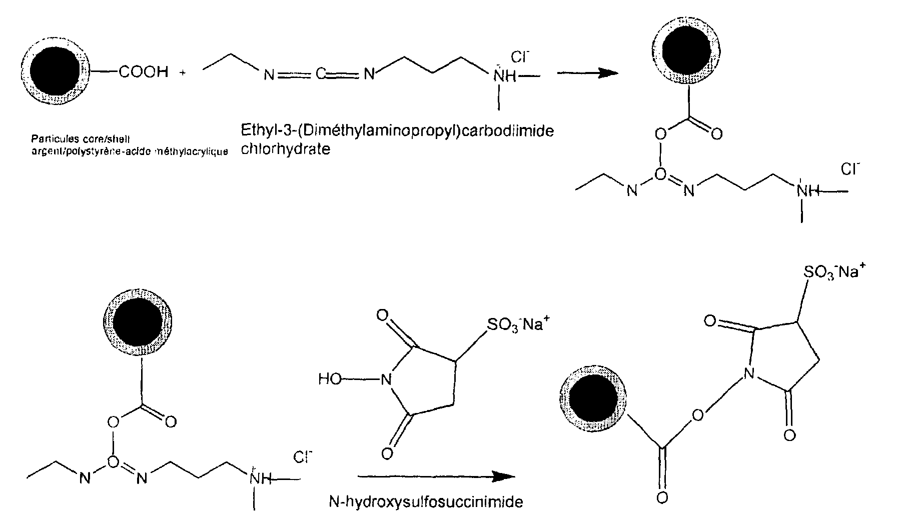

### 2- Application sur cheveux

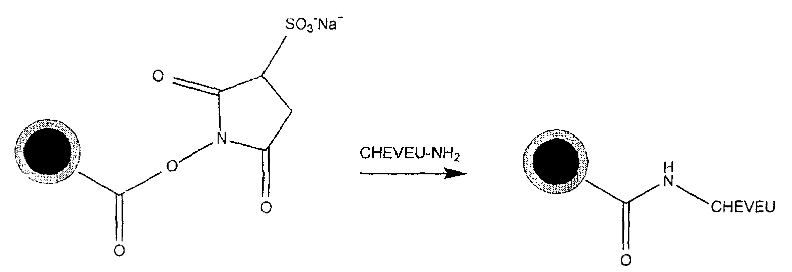

A noter que les fonctions chimiques sur la surface de la matière kératinique par exemple de la fibre capillaire peuvent être densifiées par un pré-traitement de la fibre par une solution de polymère ayant une affinité particulière pour la fibre et présentant des fonctions réactives. Dans le précédent exemple, la densité des fonctions amines à la surface de la fibre peut être augmentée, par exemple, en absorbant préalablement de la polyéthylèneimine.

Afin d'accroître la durabilité de l'effet dans le temps, outre l'amélioration de l'adhésion ou de l'adsorption, il est possible d'utiliser des particules métalliques -qui selon l'invention sont stabilisées par un colloïde protecteur- encapsulées par une enveloppe en polymère organique ou organominéral réactif capable de créer des liaisons covalentes inter-particules après évaporation de la phase solvant.

Dans les compositions cosmétiques, les nanoparticules métalliques stabilisées et éventuellement encapsulées enrobées de l'invention sont généralement présentes à une concentration comprise entre 0,0001% et 50%, de préférence entre 0,01% et 5%, et encore plus préférentiellement entre 0,05% et 2% en masse de la masse totale de la composition.

La composition selon l'invention comprend, en outre, un milieu physiologiquement acceptable. On désigne ainsi un milieu susceptible d'être appliqué sur les fibres kératiniques et en particulier sur les cheveux d'êtres humains.

Le milieu physiologiquement acceptable de la composition comprend généralement au moins un solvant, le solvant permet notamment de véhiculer les nanoparticules métalliques stabilisées et éventuellement encapsulées. Le solvant peut être généralement choisi parmi les solvants organiques, l'eau, et leurs mélanges.

Les solvants organiques sont généralement choisis parmi les alcools aliphatiques en C₁ à C₄ tels que l'éthanol et l'isopropanol, les alcools aromatiques comme l'alcool benzylique, les polyols tels que le glycérol ou le propylèneglycol, les alcanes en particulier en C₅ à C₁₀, l'acétone, la méthyl-éthyl-cétone, l'acétate de méthyle, l'acétate de butyle, l'acétate d'alkyle, le diméthoxyéthane, le diéthoxyéthane et leurs mélanges.

Les compositions selon l'invention peuvent être conditionnées sous diverses formes et notamment dans un dispositif aérosol.

La composition selon l'invention peut alors contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Les compositions de l'invention peuvent en outre contenir des additifs cosmétiques usuels choisis parmi cette liste non exhaustive telle que les agents réducteurs, les agents oxydants, les agents épaississants, les adoucissants, les agents anti-mousse, les colorants directs ou d'oxydation, les nacres, les parfums, les peptisants, les conservateurs, les tensioactifs, anioniques ou amphothères.

La composition cosmétique de l'invention peut être une composition cosmétique de traitement en particulier une composition pour apporter de la brillance aux matières kératiniques mais sous une forme de réalisation préférée, il s'agit d'une composition cosmétique capillaire en particulier d'une composition pour apporter aux cheveux de la brillance.

Les compositions cosmétiques capillaires de l'invention après application sur la chevelure peuvent être rincées ou non. Les compositions notamment capillaires (formulations) peuvent se présenter sous différentes formes galéniques tels qu'une lotion, un "spray", une mousse, une laque, un après-shampooing ou un shampooing par exemple.

L'invention sera mieux comprise à l'aide des exemples illustratifs non limitatifs qui suivent et qui constituent des modes de mise en oeuvre préférentiel de la composition de l'invention. Les pourcentages des exemples sont exprimés en poids et m.a. signifie matière active.

### Exemple comparatif

Dans cet exemple, on réalise une composition selon la présente invention, et une composition selon l'art antérieur.

### Composition 1 : Mousse aérosol selon l'invention

| | |
|---|---|
| STARCH ACETATE | 5 % m.a. |
| POLYSORBATE 20 | 0.1 % m.a. |
| COCAMIDOPROPYL BETAINE | 0.5% m.a. |
| Nanoparticules d'argent stabilisées ^{[1]} | 1,0% m.a. |
| LAURETH-4 | 0,3% m.a. |
| ISOBUTANE/BUTANE/PROPANE | 5% m.a. |
| Conservateur | qs |
| Parfum | qs |
| Eau | qsp |

- POLYSORBATE 20 : Monolaurate de polyoxyéthylène 20 sorbitan vendu par la société Atlas.
- Tensioactif LAURETH-4 : commercialisé par la société Uniquema.
- Mélange butane-isobutane-propane : 24- 56- 20.

**[1]** Les nanoparticules d'argent utilisées sont réalisées suivant la synthèse décrite dans l'exemple n°3 du document JP-A-11 080647. Tels que le montrent les clichés de microscopie électronique à transmission (TEM) réalisés, les nanoparticules ainsi réalisées présentent un diamètre compris entre 5 et 30 nm.

### Composition 2 : Mousse aérosol témoin non-conforme à l'invention

| | |
|---|---|
| STARCH ACETATE | 5 % m.a. |
| POLYSORBATE 20 | 0.1 % m.a. |
| COCAMIDOPROPYL BETAINE | 0.5% m.a. |
| Nano-particules d'argent ^{[2]} | 1,0% m.a. |
| LAURETH-4 | 0,3% m.a. |
| ISOBUTANEBUTANE/PROPANE | 5% m.a. |
| Conservateur | qs |
| Parfum | qs |
| Eau | qsp |

- POLYSORBATE 20 : Monolaurate de polyoxyéthylène 20 sorbitan vendu par la société Atlas.
- Tensioactif LAURETH-4 : commercialisé par la société Uniquema.
- Mélange butane-isobutane-propane : 24- 56- 20.

**[2]** Nanoparticules d'argent commercialisées sous la référence "Colloid Mag" par la société Grant Industries. Tels que le montrent les clichés de microscopie électronique à transmission (TEM) réalisés, les nanoparticules présentent un diamètre compris entre 5 nm et 30 nm.

Chacune des précédentes compositions est appliquée sur une mèche de cheveux châtains de 2,7 g (cheveux européens de 20 cm de longueur) à raison d'un gramme de composition par mèche. Après application, les mèches sont séchées au casque (70°C) pendant 30 minutes.

On réalise ensuite une mesure de brillance sur un lot de 10 mèches traitées comme indiqué précédemment par l'une ou l'autre des compositions.

La brillance est déterminée à l'aide un photogoniomètre par mesure des réflexions spéculaire et diffuse de mèches de cheveux posées à plat sur support. A l'aide d'une lampe xénon à arc de 175Watts (modèle ORC175F) couplé à un filtre V (lambda), une lumière est émise sur la mèche sous un angle de +30° par rapport à la normale de sa surface. A l'aide d'un bras de réception mobile on mesure la réflexion spéculaire (R) correspondant au maximum d'intensité lumineuse réfléchie aux environs de -30° d'angle et la réflexion diffuse (D) correspondant à la lumière réfléchie sous un angle de +15°. Selon l'invention, la brillance est déterminée en calculant le rapport (R)/(D)

Afin d'évaluer la tenue de la brillance dans le temps, la mesure de brillance est renouvelée sur les mêmes mèches traitées après 1 mois de stockage des ces dernières à l'ambiante (20°C et 50% HR).

Les résultats obtenus en terme de stabilité de la brillance sont donnés dans le tableau III suivant :

**Tableau III**

| | Brillance avant traitement | Brillance juste après traitement | Brillance des mèches traitées après 1 mois |
|---|---|---|---|
| Composition 1 (invention) | 22 ± 2 | 38 ± 2 | 32 ± 4 |
| Composition 2 (art antérieur) | 26 ± 2 | 36 ± 3 | 25 ± 1 |

Comme le montre le précédent tableau III, on note que la composition de l'invention conserve ces propriétés de brillance après un mois de stockage des mèches à l'ambiante. Comparativement, la composition de l'art antérieur perd la totalité de son pouvoir réflecteur après 1 mois de stockage des mèches à l'ambiante.

## Revendications

1. Composition cosmétique comprenant, dans un milieu physiologiquement acceptable, au moins un agent présentant une activité cosmétique et des particules, lesdites particules étant constituées en majorité par au moins un métal, lesdites particules étant des particules stabilisées traitées par au moins un colloïde protecteur choisi parmi les polymères peignes comprenant une chaîne principale et des chaînes latérales reliées à la chaîne principale, et la taille desdites particules étant inférieure ou égale à 500 nm.

2. Composition selon la revendication 1, dans laquelle l'agent (ou les agents) présentant une activité cosmétique est(sont) choisi(s) parmi :
• les saccharides, les oligosaccharides, les polysaccharides hydrolysés ou non, modifiés ou non,
• les acides aminés, les oligopeptides, les peptides, les protéines hydrolysées ou non, modifiées ou non, les polyacides aminés, les enzymes,
• les acides et alcools gras ramifiés ou non,
• les cires animales, végétales ou minérales,
• les céramides et les pseudo-céramides,
• les acides organiques hydroxylés,
• les filtres UV,
• les antioxydants et les agents anti-radicaux libres,
• les chélatants,
• les agents antipelliculaires,
• les agents régulateurs de séborrée,
• les agents apaisants,
• les tensioactifs cationiques,
• les polymères cationiques, amphotères,
• les silicones organomodifiées ou non,
• les huiles minérales, végétales ou animales,
• les polyisobutènes et poly(α-oléfines),
• les esters,
• les polymères anioniques solubles ou dispersés,
• les polymères non-ioniques solubles ou dispersés,
• les agents réducteurs
• les agents colorants et matières colorantes, et notamment les colorants capillaires,
• les agents moussants,
• les agents filmogènes,
• les particules, autres que les particules stabilisées définies dans la revendication 1,
• et leurs mélanges.

3. Composition selon l'une quelconque des revendications précédentes, dans laquelle l'agent présentant une activité cosmétique est présent dans une proportion de 0,001 à 10% en poids de la composition cosmétique, de préférence de 0,01 à 5% en poids.

4. Composition cosmétique selon l'une quelconque des revendications précédentes, dans laquelle la taille desdites particules est comprise entre 1 nm et 500 nm, de préférence entre 1 nm et 100 nm, et de préférence encore entre 1 nm et 50 nm.

5. Composition cosmétique selon l'une quelconque des revendications précédentes, dans laquelle les particules ont la forme de sphères, lamelles, fibres, tubes, polyèdres ou une forme aléatoire.

6. Composition cosmétique selon l'une quelconque des revendications précédentes, dans laquelle la particule est constituée par au moins 80% en masse d'au moins un métal, de préférence par au moins 90% en masse, et de préférence encore par 100% en masse d'au moins un métal.

7. Composition cosmétique selon l'une quelconque des revendications précédentes, dans laquelle le métal qui constitue en majorité les particules est choisi parmi l'aluminium et les éléments de numéro atomique allant de 21 à 82 et composant les colonnes 3 à 13 de la classification périodique des éléments, et les alliages de ceux-ci.

8. Composition cosmétique selon la revendication 7, dans laquelle les particules sont constituées par un mélange de deux ou plus desdits métaux et/ou alliages de ceux-ci.

9. Composition cosmétique selon l'une quelconque des revendications précédentes, dans laquelle les particules sont des particules composites constituées de plusieurs zones, des zones adjacentes étant constituées par des métaux, alliages ou mélanges différents.

10. Composition cosmétique selon la revendication 9, dans laquelle les particules sont des particules composites multicouches comprenant un coeur ou noyau interne constitué par un métal, alliage ou mélange de métaux ou d'alliages, recouvert au moins partiellement par une première couche en un métal, alliage de métal ou mélange de métaux ou d'alliages différent de celui constituant le coeur ou noyau interne, et éventuellement par une ou plusieurs autres couches, chacune de ces couches recouvrant au moins partiellement la couche précédente et chacune de ces couches étant constituée par un métal, alliage ou mélange différent de la couche suivante et de la couche précédente.

11. Composition selon l'une quelconque des revendications précédentes, dans laquelle les particules comprennent en outre des impuretés inévitables, et des stabilisants.

12. Composition selon l'une quelconque des revendications précédentes, dans laquelle les particules comprennent en outre des composés métalliques autres que des métaux tels que des oxydes de métaux.

13. Composition selon l'une quelconque des revendications précédentes, dans laquelle le métal qui constitue en majorité les particules est choisi parmi les métaux de transition, les métaux des terres rares et leurs alliages et mélanges.

14. Composition selon l'une quelconque des revendications précédentes, dans laquelle le métal qui constitue en majorité les particules est choisi parmi l'aluminium, le cuivre, l'argent, l'or, l'indium, le fer, le platine, le nickel, le molybdène, le titane, le tungstène, l'antimoine, la palladium, le zinc, l'étain, et leurs alliages et mélanges.

15. Composition selon la revendication 14, dans laquelle le métal qui constitue en majorité les particules est choisi parmi l'or, l'argent, le palladium, le platine et leurs alliages et mélanges.

16. Composition selon la revendication 15, dans laquelle le métal est l'argent.

17. Composition selon l'une quelconque des revendications précédentes, dans laquelle le polymère peigne avec lequel sont traitées les particules a une chaîne principale ou squelette choisie parmi les polyalkylène-imines.

18. Composition selon l'une quelconque des revendications précédentes, dans laquelle les chaînes latérales ou greffons du polymère peigne sont choisies parmi les chaînes latérales ou greffons présentant de l'affinité avec le milieu physiologiquement acceptable, de préférence les chaînes latérales ou greffons sont hydrophiles.

19. Composition selon la revendication 18, dans laquelle les chaînes latérales ou greffons sont choisies parmi les poly(esters), les polyuréthanes, les acides poly(méth)acryliques, les poly(méth)acrylates et tous les homopolymères ou copolymères présentant de l'affinité avec le milieu physiologiquement acceptable.

20. Composition selon l'une quelconque des revendications précédentes, dans laquelle les particules stabilisées sont susceptibles d'être préparées par réduction d'un ou plusieurs composés métalliques précurseurs du métal ou des métaux constituant la particule, en présence du polymère peigne.

21. Composition selon la revendication 20, dans laquelle la réduction est effectuée dans un solvant, de préférence un solvant organique et/ou de l'eau dans lequel on dissout le ou les composé(s) métallique(s) précurseur(s) et auquel on ajoute le polymère peigne puis un réducteur.

22. Composition selon l'une quelconque des revendications 20 et 21, dans laquelle le(s) composé(s) métallique(s) précurseur(s) est (sont) choisi(s) parmi l'acide chloroaurique, le nitrate d'argent, l'acétate d'argent, l'acide d'argent perchlorique, l'acide chloroplatinique, et le chloroplatinate de potassium.

23. Composition selon l'une quelconque des revendications 21 et 22, dans laquelle le réducteur est choisi parmi les réducteurs doux, par exemple parmi les molécules possédant une amine secondaire ou une amine tertiaire telle que le 2-méthylaminoéthanol, la diéthanolamine, la diéthylméthylamine, le 2-diméthylaminoéthanol, et la méthyldiéthanolamine.

24. Composition selon l'une quelconque des revendications 1 à 23, dans laquelle les particules sont des particules enrobées comprenant un coeur et une enveloppe solide, ledit coeur étant constitué par une particule telle que définie dans l'une quelconque des revendications 1 à 23, constituée en majorité par un métal, et étant traitée par au moins un colloïde protecteur choisi parmi les polymères peignes.

25. Composition selon la revendication 24, dans laquelle l'enveloppe est en un matériau organique ou inorganique lié au coeur par une liaison non covalente.

26. Composition selon la revendication 25, dans laquelle le matériau organique qui constitue l'enveloppe des particules est choisi parmi les polymères et oligomères organiques.

27. Composition selon la revendication 26, dans laquelle le matériau organique qui constitue l'enveloppe des particules est choisi parmi les poly(alcool vinylique), les poly(acide lactique), les poly(acide glycolique), les copolymères de l'acide lactique et de l'acide glycolique, les polystyrènes, les poly(méth)acrylates de méthyle, les copolymères acryliques et méthacryliques, les polyamides, les polyesters, les polyuréthanes, et les polyurées.

28. Composition selon la revendication 26, dans laquelle le matériau organique qui constitue l'enveloppe des particules est choisi parmi la cellulose et les dérivés de celle-ci comme les alkyl et hydroxyalkylcelluloses telles que la méthylcellulose, et l'éthylcellulose, et l'hydroxyalkylcellulose ou comme les esters de cellulose tels que l'acétophtalate de cellulose.

29. Composition selon la revendication 26, dans laquelle le matériau organique qui constitue l'enveloppe des particules est choisi parmi la gélatine, la pectine, éventuellement réticulées par exemple avec le glutaraldéhyde ou un polysaccharide tel que le carraghénanne.

30. Composition selon la revendication 27, dans laquelle le matériau organique qui constitue l'enveloppe des particules est un copolymère styrène-acide méthacrylique.

31. Composition selon la revendication 25, dans laquelle l'enveloppe des particules est constituée par un matériau inorganique choisi parmi les oxydes métalliques et les polymères organométalliques.

32. Composition selon l'une quelconque des revendications 25 à 31, dans laquelle l'enveloppe des particules a une épaisseur de 2 à 300 nm, de préférence de 5 à 250 nm, de préférence encore de 10 à 100 nm.

33. Composition selon l'une quelconque des revendications 25 à 32, dans laquelle la surface externe des particules est modifiée de façon covalente par au moins un groupement chimique qui est susceptible d'améliorer l'adsorption des particules sur les matières kératiniques telles que les cheveux.

34. Composition selon l'une quelconque des revendications 25 à 32, dans laquelle la surface externe des particules est modifiée de façon covalente par au moins un groupement chimique susceptible de réagir chimiquement avec les matières kératiniques telles que les cheveux.

35. Composition selon la revendication 34, dans laquelle le groupement chimique susceptible d'améliorer l'adsorption des particules sur les matières kératiniques est choisi parmi les groupements suivants :
- Acides carboxyliques et leurs sels,
- Amines primaires, secondaires, tertiaires et quaternaires,
- Phosphates,
- Oxydes de soufre tels que sulfones, sulfoniques, sulfoxides, et sulfates,
- Noyaux aromatiques tels que phényle, triazine, thiophène et imidazole.

36. Composition selon la revendication 34, dans laquelle le groupement chimique susceptible de réagir chimiquement avec les matières kératiniques est choisi parmi les groupements suivants :
- Epoxydes,
- Vinyle et vinyle activé : acrylonitrile, esters acrylique et méthacrylique, acide et esters crotonique, acide et esters cinnamique, styrène et dérivés, butadiène, éthers de vinyle, vinyle cétone, esters maléique, maléimides, vinyl sulfones, ...
- Acides carboxyliques et leurs dérivés : anhydride, chlorure d'acide, esters,
- Acétals, hémi-acétals,
- Aminals, hémi-aminals,
- Cétones et alpha-hydroxycétones, alpha-halocétones,
- Lactones, thiolactones,
- Isocyanates,
- Thiocyanates,
- Imines,
- Imides tels que succinimides, et glutimides,
- Pyridyldithio,
- N hydroxysuccinimide esters,
- Imidates,
- Oxazine et oxazoline,
- Oxazinium et oxazolinium,
- Groupements de formule R₁X, dans laquelle R₁ est un groupe alkyle de 1 à 30C, un groupe aryle comprenant de 6 à 30C, un groupe aralkyle de 7 à 30C (groupe alkyle de 1 à 30C), et X est un groupe partant tel que I, Br, Cl, OSO₃R où R est H, un groupe alkyle de 1 à 30C, -SO₂R' où R' est H, un groupe alkyle de 1 à 30C, un groupe tosyle, N(R")₃ où R" est un groupe alkyle de 1 à 30C, OPO₃R'''₂ où R'" est H ou un groupe alkyle 1 à 30C, les groupements de formule R₁X préférés sont les halogénures d'alkyle, d'aryle ou d'aralkyle.
- Groupements de formule R₂X où R₂ représente un cycle carboné de 3 à 30C ou un hétérocycle insaturé de 3 à 20 chaînons comprenant un ou plusieurs hétéroatomes choisis parmi N, S, O et P, et X est un groupe partant tel que défini ci-dessus, tels que la chlorotriazine, la chloropyrimidine, la chloroquinoxaline, le chlorobenzotriazole,
- Groupements de formule : R₃SO₂X où R₃ a la même signification que R₁ et X représente un groupe partant et a la signification déjà donnée ci-dessus,
- Lactones,
- Thiolactones,
- Siloxanes.

37. Composition selon l'une quelconque des revendications précédentes, dans laquelle les particules sont présentes dans la composition à une concentration comprise entre 0,0001% et 50%, de préférence entre 0,01 et 5% et de préférence encore entre 0,05% et 2% en masse de la masse totale de la composition.

38. Composition selon l'une quelconque des revendications précédentes, dans laquelle le milieu physiologiquement acceptable comprend au moins un solvant volatil.

39. Composition selon la revendication 38 dans laquelle le solvant volatil est choisi parmi les solvants organiques volatils, l'eau, et leurs mélanges.

40. Composition selon la revendication 39, dans laquelle les solvants organiques sont choisis parmi les alcools aliphatiques en C₁ à C₄ tels que l'éthanol et l'isopropanol, les polyols comme le glycérol ou le propylèneglycol, les alcools aromatiques comme l'alcool benzylique, les alcanes en particulier en C₅ à C₁₀, l'acétone, la méthyl-éthyl-cétone, l'acétate de méthyle, l'acétate de butyle, l'acétate d'alkyle, le diméthoxyéthane, le diéthoxyéthane et leurs mélanges.

41. Composition selon l'une quelconque des revendications précédentes qui contient en outre des additifs cosmétiques choisis parmi les agents réducteurs, les agents oxydants, les agents épaississants, les adoucissants, les agents anti-mousse, les colorants directs ou d'oxydation, les nacres, les parfums, les peptisants, les conservateurs, les tensioactifs, anioniques ou amphotères.

42. Composition selon l'une quelconque des revendications précédentes, qui est une composition cosmétique de traitement en particulier pour apporter de la brillance aux matières kératiniques.

43. Composition selon la revendication 42, qui est une composition capillaire en particulier pour apporter aux cheveux de la brillance.

44. Composition selon la revendication 43, qui se présente sous la forme d'une lotion, d'un "spray", d'une mousse, d'une laque, d'un après-shampooing ou d'un shampooing.

45. Composition selon l'une quelconque des revendications précédentes qui est conditionnée dans un dispositif aérosol.

46. Particules enrobées comprenant un coeur et une enveloppe solide telles que définies dans l'une quelconque des revendications 24 à 36.

47. Procédé cosmétique de maquillage ou de soin des matières kératiniques telles que les cheveux en particulier pour apporter de la brillance aux matières kératiniques telles que les cheveux, comprenant l'application sur des matières kératiniques telles que les cheveux d'une composition selon l'une quelconque des revendications 1 à 45.

48. Utilisation de la composition selon l'une quelconque des revendications 1 à 45, pour apporter de la brillance à des matières kératiniques telles que les cheveux.

49. Utilisation des particules selon la revendication 46, ou telles que décrites dans l'une quelconque des revendications 1 à 23 dans une composition cosmétique pour apporter de la brillance aux matières kératiniques telles que les cheveux.

## Claims

1. Cosmetic composition comprising, in a physiologically acceptable medium, at least one agent exhibiting a cosmetic activity and particles, the said particles being predominantly composed of at least one metal, the said particles being stabilized particles treated with at least one protective colloid chosen from comb polymers comprising a main chain and side chains connected to the main chain, and the size of the said particles being less than or equal to 500 nm.

2. Composition according to Claim 1, in which the agent (or agents) exhibiting a cosmetic activity is(are) chosen from:
• saccharides, oligosaccharides or polysaccharides which may or may not be hydrolysed and which may or may not be modified,
• amino acids, oligopeptides, peptides, proteins, which may or may not be hydrolysed and which may or may not be modified, poly(amino acid)s or enzymes,
• branched or unbranched fatty acids and alcohols,
• animal, vegetable or mineral waxes,
• ceramides and pseudoceramides,
• hydroxylated organic acids,
• UV screening agents,
• antioxidants and agents for combating free radicals,
• chelating agents,
• antidandruff agents,
• seborrhoea-regulating agents,
• soothing agents,
• cationic surfactants,
• cationic or amphoteric polymers,
• organomodified or nonorganomodified silicones,
• mineral, vegetable or animal oils,
• polyisobutenes and poly(α-olefin)s,
• esters,
• soluble or dispersed anionic polymers,
• soluble or dispersed non-ionic polymers,
• reducing agents,
• colouring agents and colouring materials, and in particular hair dyes,
• foaming agents,
• film-forming agents,
• particles other than the stabilized particles defined in Claim 1,
• and their mixtures.

3. Composition according to either one of the preceding claims, in which the agent exhibiting a cosmetic activity is present in a proportion of 0.001 to 10% by weight of the cosmetic composition, preferably of 0.01 to 5% by weight.

4. Cosmetic composition according to any one of the preceding claims, in which the size of the said particles is between 1 nm and 500 nm, preferably between 1 nm and 100 nm and more preferably between 1 nm and 50 nm.

5. Cosmetic composition according to any one of the preceding claims, in which the particles have the shape of spheres, flakes, fibres, tubes or polyhedra or a random shape.

6. Cosmetic composition according to any one of the preceding claims, in which the particle is composed of at least 80% by weight of at least one metal, preferably of at least 90% by weight and more preferably of 100% by weight of at least one metal.

7. Cosmetic composition according to any one of the preceding claims, in which the metal of which the particles are predominantly composed is chosen from aluminium and the elements with an atomic number ranging from 21 to 82 and composing Groups 3 to 13 of the Periodic Table of the Elements, and the alloys of the latter.

8. Cosmetic composition according to Claim 7, in which the particles are composed of a mixture of two or more of the said metals and/or alloys of the latter.

9. Cosmetic composition according to any one of the preceding claims, in which the particles are composite particles composed of several regions, adjacent regions being composed of different metals, alloys or mixtures.

10. Cosmetic composition according to Claim 9, in which the particles are multilayer composite particles comprising an inner core composed of a metal, alloy or mixture of metals or alloys, at least partially covered by a first layer made of a metal, metal alloy or mixture of metals or alloys which is different from that constituting the inner core and optionally by one or more other layers, each of these layers at least partially covering the preceding layer and each of these layers being composed of a metal, alloy or mixture which is different from the following layer and from the preceding layer.

11. Composition according to any one of the preceding claims, in which the particles additionally comprise stabilizers and unavoidable impurities.

12. Composition according to any one of the preceding claims, in which the particles additionally comprise metal compounds other than metals, such as metal oxides.

13. Composition according to any one of the preceding claims, in which the metal of which the particles are predominantly composed is chosen from transition metals, rare earth metals and their alloys and mixtures.

14. Composition according to any one of the preceding claims, in which the metal of which the particles are predominantly composed is chosen from aluminium, copper, silver, gold, indium, iron, platinum, nickel, molybdenum, titanium, tungsten, antimony, palladium, zinc, tin and their alloys and mixtures.

15. Composition according to Claim 14, in which the metal of which the particles are predominantly composed is chosen from gold, silver, palladium, platinum and their alloys and mixtures.

16. Composition according to Claim 15, in which the metal is silver.

17. Composition according to any one of the preceding claims, in which the comb polymer with which the particles are treated has a skeleton or mean chain chosen from polyalkyleneimines.

18. Composition according to any one of the preceding claims, in which the grafts or side chains of the comb polymer are chosen from grafts or side chains exhibiting affinity with the physiologically acceptable medium, preferably, the grafts or side chains are hydrophilic.

19. Composition according to Claim 18, in which the grafts or side chains are chosen from poly(esters), polyurethanes, poly((meth)acrylic acid)s, poly(meth)-acrylates and any homopolymer or copolymer exhibiting affinity with the physiologically acceptable medium.

20. Composition according to any one of the preceding claims, in which the stabilized particles are capable of being prepared by reduction, in the presence of the comb polymer, of one or more metal compounds which are precursors of the metal or metals constituting the particle.

21. Composition according to Claim 20, in which the reduction is carried out in a solvent, preferably an organic solvent and/or water, in which the precursor metal compound(s) is(are) dissolved and to which the comb polymer and then a reducing agent are added.

22. Composition according to either one of Claims 20 and 21, in which the precursor metal compound(s) is(are) chosen from chloroauric acid, silver nitrate, silver acetate, silver perchlorate, chloroplatinic acid and potassium chloroplatinate.

23. Composition according to either one of Claims 21 and 22, in which the reducing agent is chosen from mild reducing agents, for example from molecules having a secondary amine or a tertiary amine, such as 2-methylaminoethanol, diethanolamine, diethylmethylamine, 2-dimethylaminoethanol and methyldiethanolamine.

24. Composition according to any one of Claims 1 to 23, in which the particles are coated particles comprising a core and a solid shell, said core being composed of a particle as defined in any one of Claims 1 to 23, which is predominantly composed of a metal and which is treated with at lest one protective colloid chosen from comb polymers.

25. Composition according to Claim 24, in which the shell is made of an organic or inorganic material bonded to the core by a noncovalent bond.

26. Composition according to Claim 25, in which the organic material of which the shell of the particles is composed is chosen from organic polymers and oligomers.

27. Composition according to Claim 26, in which the organic material of which the shell of the particles is composed is chosen from poly(vinyl alcohol)s, poly(lactic acid)s, poly(glycolic acid)s, copolymers of lactic acid and of glycolic acid, polystyrenes, poly (methyl (meth)acrylate)s, acrylic and methacrylic copolymers, polyamides, polyesters, polyurethanes and polyureas.

28. Composition according to Claim 26, in which the organic material of which the shell of the particles is composed is chosen from cellulose and the derivatives of the latter, such as alkyl- and hydroxyalkylcelluloses, for example methylcellulose, ethylcellulose and hydroxyalkylcellulose, or such as cellulose esters, for example cellulose acetate phthalate.

29. Composition according to Claim 26, in which the organic material of which the shell of the particles is composed is chosen from gelatin, pectin, optionally crosslinked, for example with glutaraldehyde, or a polysaccharide, such as carrageenan.

30. Composition according to Claim 27, in which the organic material of which the shell of the particles is composed is a styrene-methacrylic acid copolymer.

31. Composition according to Claim 25, in which the shell of the particles is composed of an inorganic material chosen from metal oxides and organometallic polymers.

32. Composition according to any one of Claims 25 to 31, in which the shell of the particles has a thickness of 2 to 300 nm, preferably of 5 to 250 nm, more preferably of 10 to 100 nm.

33. Composition according to any one of Claims 25 to 32, in which the outer surface of the particles is covalently modified by at least one chemical group which is capable of improving the adsorption of the particles on keratinous substances, such as the hair.

34. Composition according to any one of Claims 25 to 32, in which the outer surface of the particles is covalently modified by at least one chemical group capable of reacting chemically with keratinous substances, such as the hair.

35. Composition according to Claim 34, in which the chemical group capable of improving the adsorption of the particles on keratinous substances is chosen from the following groups:
- Carboxylic acids and their salts,
- Primary, secondary, tertiary and quaternary amines,
- Phosphates,
- Sulphur oxides, such as sulphones, sulphonic acids, sulphoxides and sulphates,
- Aromatic rings, such as phenyl, triazine, thiophene and imidazole.

36. Composition according to Claim 34, in which the chemical group capable of reacting chemically with keratinous substances is chosen from the following groups:
- Epoxides,
- Vinyl and activated vinyl: acrylonitrile, acrylic and methacrylic esters, crotonic acid and esters, cinnamic acid and esters, styrene and derivatives, butadiene, vinyl ethers, vinyl ketones, maleic esters, maleimides, vinyl sulphones, and the like,
- Carboxylic acids and their derivatives: anhydride, acid chloride, esters,
- Acetals, hemiacetals,
- Aminals, hemiaminals,
- Ketones and α-hydroxyketones, α-haloketones,
- Lactones, thiolactones,
- Isocyanates,
- Thiocyanates,
- Imines,
- Imides, such as succinimides and glutimides,
- Pyridyldithio,
- N-Hydroxysuccinimide esters,
- Imidates,
- Oxazine and oxazoline,
- Oxazinium and oxazolinium,
- Groups of formula R₁X in which R₁ is an alkyl group of 1 to 30C, an aryl group comprising from 6 to 30C or an aralkyl group of 7 to 30C (alkyl group of 1 to 30C) and X is a leaving group such as I, Br, Cl, OSO₃R, where R is H or an alkyl group of 1 to 30C, -SO₂R*'*, where R' is H or an alkyl group of 1 to 30C, a tosyl group, N(R*"*)₃, where R" is an alkyl group of 1 to 30C, or OPO₃R"'₂, where R"' is H or an alkyl group of 1 to 30C; preferred groups of formula R₁X are alkyl, aryl or aralkyl halides,
- Groups of formula R₂X where R₂ represents a carbon ring of 3 to 30C or an unsaturated heterocycle with 3 to 20 ring members comprising one or more heteroatoms chosen from N, S, O and P, and X is a leaving group as defined above, such as chlorotriazine, chloropyrimidine, chloroquinoxaline or chlorobenzotriazole,
- Groups of formula R₃SO₂X, where R₃ has the same meaning as R₁ and X represents a leaving group and has the meaning already given above,
- Lactones,
- Thiolactones,
- Siloxanes.

37. Composition according to any one of the preceding claims, in which the particles are present in the composition at a concentration of between 0.0001% and 50%, preferably between 0.01% and 5% and more preferably between 0.05% and 2% by weight of the total weight of the composition.

38. Composition according to any one of the preceding claims, in which the physiologically acceptable medium comprises at least one volatile solvent.

39. Composition according to Claim 38, in which the volatile solvent is chosen from volatile organic solvents, water and their mixtures.

40. Composition according to Claim 39, in which the organic solvents are chosen from C₁ to C₄ aliphatic alcohols, such as ethanol and isopropanol, polyols, such as glycerol or propylene glycol, aromatic alcohols, such as benzyl alcohol, alkanes, in particular C₅ to C₁₀ alkanes, acetone, methyl ethyl ketone, methyl acetate, butyl acetate, alkyl acetate, dimethoxyethane, diethoxyethane and their mixtures.

41. Composition according to any one of the preceding claims, which additionally comprises cosmetic additives chosen from reducing agents, oxidizing agents, thickening agents, softeners, antifoaming agents, direct or oxidation dyes, pearlescent agents, fragrances, peptizing agents, preservatives, or anionic or amphoteric surfactants.

42. Composition according to any one of the preceding claims, which is a cosmetic treatment composition, in particular for contributing sheen to keratinous substances.

43. Composition according to Claim 42, which is a hair composition, in particular for contributing sheen to the hair.

44. Composition according to Claim 43, which is provided in the form of a lotion, of a spray, of a foam, of a lacquer, of a conditioner or of a shampoo.

45. Composition according to any one of the preceding claims, which is packaged in an aerosol device.

46. Coated particles comprising a core and a solid shell such as are defined in any one of Claims 24 to 36.

47. Cosmetic process for making up or caring for keratinous substances, such as the hair, in particular for contributing sheen to keratinous substances, such as the hair, comprising the application to keratinous substances, such as the hair, of a composition according to any one of Claims 1 to 45.

48. Use of the composition according to any one of Claims 1 to 45 for contributing sheen to keratinous substances, such as the hair.

49. Use of the particles according to Claim 23 or as described in any one of Claims 1 to 23 in a cosmetic composition for contributing sheen to keratinous substances, such as the hair.

## Patentansprüche

1. Kosmetische Zusammensetzung, die in einem physiologisch akzeptablen Medium mindestens einen Stoff, der eine kosmetische Wirksamkeit aufweist, und Partikel enthält, wobei die Partikel in einem überwiegenden Anteil aus mindestens einem Metall bestehen, wobei die Partikel stabilisierte Partikel sind, die mit mindestens einem Schutzkolloid behandelt wurden, das unter den Kammpolymeren ausgewählt ist, die eine Hauptkette und an die Hauptkette gebundene Seitenketten aufweisen, und wobei die Größe der Partikel höchstens 500 nm beträgt.

2. Zusammensetzung nach Anspruch 1, wobei der Stoff (oder die Stoffe) mit kosmetischer Wirksamkeit ausgewählt ist (sind) unter:
• Sacchariden, Oligosacchariden, Polysacchariden, die hydrolysiert oder nichthydrolysiert, modifiziert oder nichtmodifiziert sind,
• Aminosäuren, Oligopeptiden, Peptiden, Proteinen, die gegebenenfalls hydrolysiert, gegebenenfalls modifiziert sind, Polyaminosäuren, Enzymen,
• verzweigten oder unverzweigten Fettsäuren und Fettalkoholen,
• tierischen, pflanzlichen oder mineralischen Wachsen,
• Ceramiden und Pseudoceramiden,
• hydroxylierten organischen Säuren,
• UV-Filtern,
• Antioxidantien und Radikalfängern für freie Radikale,
• Chelatbildnern,
• Antischuppenmitteln,
• die Seborrhoe regulierenden Stoffen,
• beruhigenden Stoffen,
• kationischen grenzflächenaktiven Stoffen,
• kationischen, amphoteren Polymeren,
• organomodifizierten oder nichtorganomodifizierten Siliconen,
• Mineralölen, pflanzlichen Ölen oder tierischen Ölen,
• Polyisobutenen und Poly(α-olefinen),
• Estern,
• löslichen oder dispergierten anionischen Polymeren,
• löslichen oder dispergierten nichtionischen Polymeren,
• Reduktionsmitteln,
• Farbstoffen und Farbmitteln, insbesondere Haarfärbemitteln,
• schaumbildenden Stoffen,
• Filmbildnern,
• Partikeln, die von den in Anspruch 1 definierten stabilisierten Partikeln verschieden sind, und
• deren Gemischen.

3. Zusammensetzung nach einem der vorhergehenden Ansprüche, wobei der Stoff mit kosmetischer Wirksamkeit in einem Mengenanteil von 0,001 bis 10 Gew.-% der kosmetischen Zusammensetzung und vorzugsweise 0,01 bis 5 Gew.-% enthalten ist.

4. Kosmetische Zusammensetzung nach einem der vorhergehenden Ansprüche, wobei die Größe der Partikel im Bereich von 1 bis 500 nm, vorzugsweise 1 bis 100 nm und noch bevorzugter 1 bis 50 nm liegt.

5. Kosmetische Zusammensetzung nach einem der vorhergehenden Ansprüche, wobei die Partikel die Form von Sphären, Lamellen, Fasern, Röhren, Polyedern oder zufällige Formen haben.

6. Kosmetische Zusammensetzung nach einem der vorhergehenden Ansprüche, wobei der Partikel aus mindestens 80 Masse-% mindestens eines Metalls, vorzugsweise mindestens 90 Masse-% und noch bevorzugter 100 Masse-% mindestens eines Metalls besteht.

7. Kosmetische Zusammensetzung nach einem der vorhergehenden Ansprüche, wobei das Metall, das die Partikel hauptsächlich bildet, unter Aluminium und Elementen mit der Ordnungszahl 21 bis 82 und aus der 3. bis 13. Gruppe des Periodensystems der Elemente und ihren Legierungen ausgewählt ist.

8. Kosmetische Zusammensetzung nach Anspruch 7, wobei die Partikel aus einem Gemisch von zwei oder mehr dieser Metalle und/oder ihren Legierungen bestehen.

9. Kosmetische Zusammensetzung nach einem der vorhergehenden Ansprüche, wobei die Partikel Verbundpartikel sind, die aus mehreren Zonen bestehen, wobei aneinander angrenzende Zonen aus unterschiedlichen Metallen, Legierungen oder Gemischen bestehen.

10. Kosmetische Zusammensetzung nach Anspruch 9, wobei die Partikel mehrlagige Verbundpartikel sind, die einen Kern oder inneren Nukleus aufweisen, der aus einem Metall, einer Legierung oder einem Gemisch von Metallen oder Legierungen besteht, der zumindest teilweise von einer ersten Lage aus einem Metall, einer Metalllegierung oder einem Gemisch von Metallen oder Legierungen, die sich von dem unterscheiden, aus dem der Kern oder innere Nukleus besteht, und gegebenenfalls mit einer oder mehreren weiteren Lagen beschichtet ist, wobei jede dieser Lagen zumindest teilweise die vorhergehende Lage bedeckt und wobei jede dieser Lagen aus einem Metall, einer Legierung oder einem Gemisch besteht, das anders als das der vorhergehenden und der folgenden Schicht ist.

11. Kosmetische Zusammensetzung nach einem der vorhergehenden Ansprüche, wobei die Partikel ferner unvermeidliche Verunreinigungen und Stabilisatoren enthalten.

12. Kosmetische Zusammensetzung nach einem der vorhergehenden Ansprüche, wobei die Partikel ferner metallische Verbindungen enthalten, die von den Metallen verschieden sind, wie Metalloxide.

13. Zusammensetzung nach einem der vorhergehenden Ansprüche, wobei das Metall, das hauptsächlich die Partikel bildet, unter den Übergangsmetallen, Seltenerdmetallen und ihren Legierungen und Gemischen ausgewählt ist.

14. Zusammensetzung nach einem der vorhergehenden Ansprüche, wobei das Metall, das hauptsächlich die Partikel bildet, unter Aluminium, Kupfer, Silber, Gold, Indium, Eisen, Platin, Nickel, Molybdän, Titan, Wolfram, Antimon, Palladium, Zink, Zinn und deren Legierungen und Gemischen ausgewählt ist.

15. Zusammensetzung nach Anspruch 14, wobei das Metall, das hauptsächlich die Partikel bildet, unter Gold, Silber, Palladium, Platin und deren Legierungen und Gemischen ausgewählt ist.

16. Zusammensetzung nach Anspruch 15, wobei es sich bei dem Metall um Silber handelt.

17. Zusammensetzung nach einem der vorhergehenden Ansprüche, wobei das Kammpolymer, mit dem die Partikel behandelt sind, eine Hauptkette oder ein Grundgerüst aufweist, das unter den Polyalkyleniminen ausgewählt ist.

18. Zusammensetzung nach einem der vorhergehenden Ansprüche, wobei die Seitenketten oder Pfropfzweige des Kammpolymers unter den Seitenketten oder Pfropfzweigen ausgewählt sind, die eine Affinität für das physiologisch akzeptable Medium aufweisen, wobei die Seitenketten oder Pfropfzweige vorzugsweise hydrophil sind.

19. Zusammensetzung nach Anspruch 18, wobei die Seitenketten oder Pfropfzweige unter den Polyestern, Polyurethanen, Poly(meth)acrylsäure, Poly(meth)acrylaten und allen Homopolymeren oder Copolymeren ausgewählt sind, die eine Affinität für das physiologisch akzeptable Medium haben.

20. Zusammensetzung nach einem der vorhergehenden Ansprüche, wobei die stabilisierten Partikel durch Reduktion einer oder mehrerer metallischer Vorläuferverbindungen des Metalls oder der Metalle, die die Partikel bilden, in Gegenwart des Kammpolymers erhältlich sind.

21. Zusammensetzung nach Anspruch 20, wobei die Reduktion in einem Lösungsmittel, vorzugsweise einem organischen Lösungsmittel und/oder Wasser, durchgeführt wird, in dem man die metallische(n) Vorläuferverbindung(en) löst und zu dem das Kammpolymer und anschließend ein Reduktionsmittel gegeben wird.

22. Zusammensetzung nach einem der Ansprüche 20 und 21, wobei die metallische(n) Vorläuferverbindung(en) unter Chlorgoldsäure, Silbernitrat, Silberacetat, Perchlorsilbersäure, Chlorplatinsäure und Kaliumchlorplatinat ausgewählt ist (sind).

23. Zusammensetzung nach einem der Ansprüche 21 und 22, wobei das Reduktionsmittel unter den milden Reduktionsmitteln, beispielsweise Molekülen, die ein sekundäres Amin oder ein tertiäres Amin enthalten, wie 2-Methylaminoethanol, Diethanolamin, Diethylmethylamin, 2-Dimethylaminoethanol und Methyldiethanolamin, ausgewählt ist.

24. Zusammensetzung nach einem der Ansprüche 1 bis 23, wobei die Partikel umhüllte Partikel sind, die einen Kern und eine feste Schale aufweisen, wobei der Kern aus einem Partikel besteht, wie er in einem der Ansprüche 1 bis 23 definiert wurde, der hauptsächlich aus einem Metall besteht und mit mindestens einem Schutzkolloid behandelt wurde, das unter den Kammpolymeren ausgewählt ist.

25. Zusammensetzung nach Anspruch 24, wobei die Schale aus einem organischen oder anorganischen Material besteht, das über eine nichtkovalente Bindung an den Kern gebunden ist.

26. Zusammensetzung nach Anspruch 25, wobei das organische Material, das die Schale der Partikel bildet, unter den organischen Polymeren und Oligomeren ausgewählt ist.

27. Zusammensetzung nach Anspruch 26, wobei das organische Material, das die Schale der Partikel bildet, ausgewählt ist unter Polyvinylalkoholen, Polymilchsäuren, Polyglycolsäuren, Copolymeren von Milchsäure und Glycolsäure, Polystyrolen, Polymethyl(meth)acrylaten, Acrylcopolymeren und Methacrylcopolymeren, Polyamiden, Polyestern, Polyurethanen und Polyharnstoffen.

28. Zusammensetzung nach Anspruch 26, wobei das organische Material, das die Schale der Partikel bildet, unter Cellulose und Cellulosederivaten, wie Alkyl- und Hydroxyalkylcellulosen, beispielsweise Methylcellulose, Ethylcellulose, und Hydroxyalkylcellulosen oder Celluloseestern, wie Celluloseacetophthalat, ausgewählt ist.

29. Zusammensetzung nach Anspruch 26, wobei das organische Material, das die Schale der Partikel bildet, unter Gelatine, Pektin ausgewählt ist, die gegebenenfalls beispielsweise mit Glutaraldehyd oder einem Polysaccharid wie Carraghenan vernetzt sind.

30. Zusammensetzung nach Anspruch 27, wobei das organische Material, das die Schale der Partikel bildet, ein Styrol/Methacrylsäure-Copolymer ist.

31. Zusammensetzung nach Anspruch 25, wobei die Schale der Partikel aus einem anorganischen Material besteht, das unter den Metalloxiden und Metall-organischen Polymeren ausgewählt ist.

32. Zusammensetzung nach einem der Ansprüche 25 bis 31, wobei die Hülle der Partikel eine Dicke von 2 bis 300 nm, vorzugsweise 5 bis 250 nm und bevorzugt 10 bis 100 nm aufweist.

33. Zusammensetzung nach einem der Ansprüche 25 bis 32, wobei die äußere Oberfläche der Partikel mit mindestens einer chemischen Gruppe in kovalenter Weise modifiziert ist, die die Adsorption der Partikel auf den Keratinsubstanzen wie Haaren verbessern kann.

34. Zusammensetzung nach einem der Ansprüche 25 bis 32, wobei die äußere Oberfläche der Partikel mit mindestens einer chemischen Gruppe in kovalenter Weise modifiziert ist, die chemisch mit Keratinsubstanzen wie Haaren reagieren kann.

35. Zusammensetzung nach Anspruch 34, wobei die chemische Gruppe, die die Adsorption der Partikel auf den Keratinsubstanzen verbessern kann, unter den folgenden Gruppen ausgewählt ist:
- Carbonsäuren und ihren Salzen,
- primären, sekundären, tertiären und quartären Aminen,
- Phosphaten,
- Schwefeloxiden, wie Sulfonen, Sulfonsäuren, Sulfoxiden und Sulfaten,
- aromatischen Kernen, wie Phenyl, Triazin, Thiophen und Imidazol.

36. Zusammensetzung nach Anspruch 34, wobei die chemische Gruppe, die mit Keratinsubstanzen chemisch reagieren kann, unter den folgenden Gruppen ausgewählt ist:
- Epoxiden,
- Vinylverbindungen und aktivierten Vinylverbindungen: Acrylnitril, Acrylsäureestern und Methacrylsäureestern, Crotonsäure und Crotonsäureestern, Zimtsäure und Zimtsäureestern, Styrol und seinen Derivaten, Butadien, Vinylethern, Vinylketon, Maleinsäureestern, Maleimiden, Vinylsulfonen, ...
- Carbonsäuren und ihren Derivaten: Anhydriden, Säurechloriden, Estern,
- Acetalen, Halbacetalen,
- Aminalen, Halbaminalen,
- Ketonen und alpha-Hydroxyketonen, alpha-Halogenketonen,
- Lactonen, Thiolactonen,
- Isocyanaten,
- Thioisocyanaten,
- Iminen,
- Imiden, wie Succinimiden und Glutimiden,
- Pyridyldithio,
- N-Hydroxysuccinimidestern,
- Imidaten,
- Oxazinen und Oxazolinen,
- Oxazinium und Oxazolinium,
- Gruppen der Formel R₁X, worin R₁ eine Alkylgruppe mit 1 bis 30 Kohlenstoffatomen, eine Arylgruppe mit 6 bis 30 Kohlenstoffatomen, eine Aralkylgruppe mit 7 bis 30 Kohlenstoffatomen (Alkylgruppe mit 1 bis 30 C) bedeutet, X eine austretende Gruppe ist, wie I, Br, Cl, OSO₃R, wobei R H, C₁₋₃₀-Alkyl bedeutet, -SO₂R', wobei R' H, C₁₋₃₀-Alkyl bedeutet, Tosyl, N(R")₃, wobei R" C₁₋₃₀-Alkyl bedeutet, OPO₃R"'₂, wobei R'" H oder C₁₋₃₀-Alkyl bedeutet, wobei bevorzugte Gruppen der Formel R₁X halogenierte Alkyle, halogenierte Aryle oder halogenierte Aralkyle sind,
- Gruppen der Formel R₂X, wobei R₂ einen Kohlenstoffring mit 3 bis 30 C oder einen 3- bis 20-gliedrigen, ungesättigten Heterocyclus bedeutet, der ein oder mehrere Heteroatome enthält, die unter N, S, O und P ausgewählt sind, und X eine austretende Gruppe ist, wie sie oben definiert wurde, beispielsweise Chlortriazin, Chlorpyrimidin, Chlorchinoxalin, Chlorbenzotriazol,
- Gruppen der Formel: R₃SO₂X, wobei R₃ die oben für R₁ angegebenen Bedeutungen aufweist und X eine austretende Gruppe ist, die die bereits oben angegebenen Bedeutungen hat,
- Lactonen,
- Thiolactonen,
- Siloxanen.

37. Zusammensetzung nach einem der vorhergehenden Ansprüche, wobei die Partikel in der Zusammensetzung in einer Konzentration von 0,0001 bis 50 %, vorzugsweise 0,01 bis 5 % und noch bevorzugter 0,05 bis 2 Gew.-% des Gesamtgewichts der Zusammensetzung enthalten sind.

38. Zusammensetzung nach einem der vorhergehenden Ansprüche, wobei das physiologisch akzeptable Medium mindestens ein flüchtiges Lösungsmittel enthält.

39. Zusammensetzung nach Anspruch 38, wobei das flüchtige Lösungsmittel unter den flüchtigen organischen Lösungsmitteln, Wasser und deren Gemischen ausgewählt ist.

40. Zusammensetzung nach Anspruch 39, wobei die organischen Lösungsmittel unter den aliphatischen C₁₋₄-Alkoholen, wie Ethanol und Isopropanol, Polyolen wie Glycerin oder Propylenglycol, aromatischen Alkoholen wie Benzylalkohol, Alkanen, insbesondere solchen mit 5 bis 10 Kohlenstoffatomen, Aceton, Methylethylketon, Methylacetat, Butylacetat, Alkylacetaten, Dimethoxyethan, Diethoxyethan und deren Gemischen ausgewählt sind.

41. Zusammensetzung nach einem der vorhergehenden Ansprüche, die ferner kosmetische Zusatzstoffe enthält, die unter den Reduktionsmitteln, Oxidationsmitteln, Verdickungsmitteln, beruhigenden Stoffen, Schaumverhütungsmitteln, Direktfarbstoffen oder Oxidationsfarbstoffen, Perlglanzpigmenten, Parfums, peptisierenden Stoffen, Konservierungsmitteln, anionischen oder amphoteren grenzflächenaktiven Stoffen ausgewählt sind.

42. Zusammensetzung nach einem der vorhergehenden Ansprüche, bei der es sich um eine kosmetische Zusammensetzung für die Behandlung handelt, insbesondere, um den Keratinsubstanzen Glanz zu geben.

43. Zusammensetzung nach Anspruch 42, die eine Zusammensetzung für die Haarbehandlung ist, insbesondere, um den Haaren Glanz zu geben.

44. Zusammensetzung nach Anspruch 43, die als Lotion, "Spray", Schaum, Lack, Conditioner oder Haarwaschmittel vorliegt.

45. Zusammensetzung nach einem der vorhergehenden Ansprüche, die in einer Aerosolvorrichtung konfektioniert ist.

46. Umhüllte Partikel, die einen Kern und eine feste Schale aufweisen, wie sie in einem der Ansprüche 24 bis 36 definiert wurden.

47. Kosmetisches Verfahren zum Schminken oder für Pflege von Keratinsubstanzen wie Haaren, insbesondere, um den Keratinsubstanzen wie Haaren Glanz zu geben, das das Aufbringen einer Zusammensetzung nach einem der Ansprüche 1 bis 45 auf Keratinsubstanzen wie Haaren, umfasst.

48. Verwendung der Zusammensetzung nach einem der Ansprüche 1 bis 45, um Keratinsubstanzen wie Haaren Glanz zu geben.

49. Verwendung von Partikeln nach Anspruch 46 oder Partikeln, wie sie in einem der Ansprüche 1 bis 23 beschrieben sind, in einer kosmetischen Zusammensetzung, um Keratinsubstanzen wie Haaren Glanz zu geben.
